(19) Europäisches Patentamt
European Patent Office
Office européen des brevets

(11) **EP 4 387 511 B1**

(12) **EUROPEAN PATENT SPECIFICATION**

(45) Date of publication and mention
of the grant of the patent:
**22.04.2026 Bulletin 2026/17**

(21) Application number: **22857157.6**

(22) Date of filing: **22.08.2022**

(51) International Patent Classification (IPC):
***A61M 16/06*** *(2006.01)*

(52) Cooperative Patent Classification (CPC):
**A61M 16/024; A61M 16/208; A61M 16/209;
G16H 20/40; G16H 40/63;** A61B 5/087;
A61M 16/0069; A61M 16/0072; A61M 16/06;
A61M 16/0672; A61M 16/0683; A61M 16/107;
A61M 16/16; A61M 2016/0027; A61M 2016/0039;
(Cont.)

(86) International application number:
**PCT/AU2022/050934**

(87) International publication number:
**WO 2023/019325 (23.02.2023 Gazette 2023/08)**

(54) **VALVE ASSEMBLY**

VENTILANORDNUNG

ENSEMBLE VALVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.08.2021 AU 2021902613
22.12.2021 AU 2021904191
18.05.2022 AU 2022901329**

(43) Date of publication of application:
**26.06.2024 Bulletin 2024/26**

(73) Proprietor: **ResMed Pty Ltd
Bella Vista, New South Wales 2153 (AU)**

(72) Inventors:
• **DEUBEL, Sebastien
Bella Vista, New South Wales 2153 (AU)**
• **FOSTER, Sophie Evelyn
Bella Vista, New South Wales 2153 (AU)**
• **HOLLEY, Liam
Bella Vista, New South Wales 2153 (AU)**
• **TRUSCOTT, Michael Kenneth
Bella Vista, New South Wales 2153 (AU)**
• **VESCHAMBRE, Etienne
Bella Vista, New South Wales 2153 (AU)**

(74) Representative: **Vossius & Partner
Patentanwälte Rechtsanwälte mbB
Siebertstrasse 3
81675 München (DE)**

(56) References cited:
EP-B1- 1 491 227        WO-A1-2011/051908
WO-A1-2021/189033       GB-A- 748 363
US-A- 3 556 122         US-A1- 2008 178 879
US-A1- 2014 144 447     US-A1- 2017 252 524
US-A1- 2020 337 634     US-A1- 2021 038 849
US-A1- 2021 038 849     US-B1- 6 634 357
US-B1- 6 634 357

(52) Cooperative Patent Classification (CPC): (Cont.)
A61M 2202/0208; A61M 2205/332;
A61M 2205/3368; A61M 2205/3375; A61M 2205/42;
A61M 2205/505; A61M 2205/7518; A61M 2230/60;
A61M 2230/63

**Description**

1 BACKGROUND OF THE TECHNOLOGY

1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

1.2 DESCRIPTION OF THE RELATED ART

**1.2.1 Human Respiratory System and its Disorders**

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO2 to meet the patient's needs. Respiratory failure may encompass some or all of the following disorders.

A patient with respiratory insufficiency (a form of respiratory failure) may experience abnormal shortness of breath on exercise.

Obesity Hypoventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 1.2.2 Therapies

Various respiratory therapies, such as Positive Airway Pressure (PAP) and in particular Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube or endotracheal tube. In some forms, the comfort and effectiveness of these therapies may be improved.

### 1.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that may be held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired $CO_2$ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched air at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 1.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 1.2.4 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

### 1.2.4.1 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH2O).

| RPT Device name | A-weighted sound pressure level dB(A) | Year (approx.) |
|---|---|---|
| C-Series TangoTM | 31.9 | 2007 |
| C-Series TangoTM with Humidifier | 33.1 | 2007 |
| S8 EscapeTM II | 30.5 | 2005 |
| S8 EscapeTM II with H4iTM Humidifier | 31.1 | 2005 |
| S9 AutoSetTM | 26.5 | 2010 |

(continued)

| RPT Device name | A-weighted sound pressure level dB(A) | Year (approx.) |
|---|---|---|
| S9 AutoSetTM with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

Typical RPT devices may consume up to 10W of power (excluding power required to operate a humidifier). For minimising the amount of consumed power, it is important to improve the efficiency of the device. While this is important if the RPT is powered from a mains power source, it is even more so if the device is run on battery, as the high power consumption may decrease the time the RPT can be run from a battery, for example if the RPT is portable, if a mains power source is unavailable (for example if the patient is camping), or if the mains power supply is interrupted.

The development of portable PAP devices, including wearable combined blower/mask systems, further necessitates the use of smaller motors/turbines and power supply/batteries. However, such smaller systems typically offer less pneumatic performance compared to a traditional PAP system. One way to maintain a relatively high pneumatic performance is to optimise the performance of such systems by minimising any wasted energy and making the systems more efficient. However, it is important to ensure that any changes in any elements of the system do not interfere with the remaining system elements and that the optimised system is still quiet, efficient, and does not cause any inconvenience or increased effort on behalf of the user.

### 1.2.4.2 Power supply

RPT devices require a power supply to operate. Power supplies add bulk and weight to a respiratory therapy system, especially if the system is operated by batteries and not from the mains. Keeping the power supply small again necessitates a higher optimisation and reduced inefficiency and wastages in the power management of the device.

### 1.2.4.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.4.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore, medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 1.2.4.5 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 1.2.4.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. In many cases, gas vents continuously though the vent at all points in the patient's respiratory cycle.

Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

### 1.2.5 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

WO 2011/051908 A1 discloses a conduit operative between a patient interface and both (a) a tidal volume gas delivery device that can deliver controlled tidal volume gasses to a patient for the purposes of resuscitation and (b) a CPAP device for delivery of CPAP to the patient.

US 6,634,357 B1 discloses a resuscitation valve assembly that includes a housing with an inlet port adapted to be connected to a pressurized breathable gas source, such as oxygen, an outlet port adapted to be connected to a patient's airway and an ambient air port. The inhalation chamber is connected to the pressurized gas source when a manually operated valve, within the housing, is open and is disconnected from the inhalation chamber when the valve is closed.

US 2021/038849 A1 discloses a vent assembly for use with a respiratory mask of the type used in CPAP treatment which includes a porous disk portion.

## 2 BRIEF SUMMARY OF THE TECHNOLOGY

The subject-matter of the present invention is defined by the features of independent claim 1. Further preferred embodiments of the present invention are defined in the dependent claims. In the following, certain aspects are disclosed which are useful for the understanding of the present invention, In particular, the present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One aspect of the present technology is a valve assembly.

Another aspect of the present technology is an air circuit comprising a valve assembly.

Another aspect of the present technology is a method of characterising vent flow in a respiratory treatment system.

Another aspect of the present technology is a method of estimating vent flows from a patient interface in the presence of an expiratory activated valve.

Another aspect of the present technology is a system for treating a respiratory disorder comprising a patient interface, an air circuit and an RPT device, wherein the air circuit comprises a valve assembly.

Another aspect of the present technology is a system for treating a respiratory disorder of a patient, the system comprising a patient interface, an air circuit and an RPT device, wherein the RPT device is controlled to decrease output upon detection of exhalation of the patient.

In examples, the system comprises a combined one way inspiratory valve and expiratory release valve between the RPT device and the patient, wherein the RPT device decreases output upon detection that a pressure on a patient side of the one way inspiratory valve and expiratory release valve exceeds a preselected pressure.

An aspect of one form of the present technology is a portable RPT device that may be carried by a person, e.g., around the home of the person.

Another aspect of the present technology comprises a combined one way inspiratory valve and expiratory release valve assembly, for controlling air flow in a respiratory treatment system for delivering pressurised air to an entrance to a patient's airways, the system being configured to maintain a therapy pressure in a range suitable for treating respiratory disorders,

the combined valve assembly comprising:

a housing comprising a valve inlet, a valve outlet and at least one vent opening,

a diaphragm sealingly connected to the housing at an outer circumference of the diaphragm, wherein the diaphragm divides the housing into; a) an upstream portion, which is in fluid communication with the valve inlet, and b) a downstream portion which is in fluid communication with the valve outlet,

wherein the diaphragm has a circular, oval, elliptical or stadium shape,
wherein an inner portion of the diaphragm defines a one way inspiratory valve configured to;

- allow flow from the valve inlet to the valve outlet when the pressure in the upstream portion of the housing exceeds the pressure in the downstream portion of the housing; and
- reduce or substantially prevent flow from the valve inlet to the valve outlet when the pressure in the downstream portion is greater than the pressure in the upstream portion,

wherein an outer portion of the diaphragm defines an expiratory release valve that,

- when the pressure in the downstream portion exceeds the pressure in the upstream portion, allows flow from the downstream portion of the housing to ambient atmosphere via the at least one vent opening; and

- when the pressure in the upstream portion exceeds the pressure in the downstream portion, reduces or substantially prevents flow from the downstream portion of the housing to ambient atmosphere via the at least one vent opening.

In examples:
a) the diaphragm has an elongate shape, and a ratio of the length of the major axis of the diaphragm to the length of the minor axis of the diaphragm is at least 4:3; b) the one-way valve formation is configured as a duckbill valve, and wherein a base of the duckbill valve has a length dimension substantially parallel to the major axis of the diaphragm and a width dimension substantially parallel to the minor axis of the diaphragm, wherein the length is greater than the width; c) the ratio of length to width is at least 1.5:1 or 2:1; d) the diaphragm comprises an outer retention flange; e) the diaphragm comprises a cylindrical wall, wherein the retention flange is provided to one end of the cylindrical wall; f) a first portion of the diaphragm has at least one material or physical property which is different from an adjacent portion of the diaphragm; g) the first portion of the diaphragm is made from a different material to the adjacent portion; h) the diaphragm is integrally formed from a single material; i) the diaphragm has regions of differing thickness; one of the regions has a thickness which is at least twice the thickness of another of the regions; j) one of the regions has a thickness which is between two and eight times the thickness of another of the regions; k) the one-way inspiratory valve has a first region having a first thickness and a second region, adjacent the first region, having a second thickness; 1) the expiratory release valve has a first region having a first thickness and a second region, adjacent the first region, having a second thickness; m) the diaphragm has no more than a single axis of symmetry; n) the at least one vent opening comprises a plurality of vent openings spaced apart around an outer periphery of the downstream portion of the housing; o) the downstream portion of the housing comprises the valve outlet, wherein the diaphragm comprise a pair of lips that are pushed towards each other to seal the pathway to the patient interface port when the pressure in the downstream portion exceeds the pressure in the upstream portion, and wherein the pair of lips create an opening to allow airflow to pass through towards the patient interface port entrance, when the pressure in the upstream portion of the housing exceeds the pressure in the downstream portion; p) a flow guide is provided for each vent opening, each flow guide configured to avoid or minimise at least one of i) sharp corners; ii) sharp angles; and iii) sudden expansion, of expiratory gases flowing from the valve outlet to the respective vent opening, when the pressure in the downstream portion exceeds the pressure in the upstream portion; q) each flow guide comprises a ramp portion guiding the exhalation airflow to the entrance to the vent opening; and/or r) each flow guide comprises side wall portions provided to either side of each respective ramp portion.

Another aspect of the present technology comprises a patient interface system for providing an airflow generated by a blower to a patient, the patient interface system comprising the valve of any one of the preceding paragraphs.

Another aspect of the present technology comprises a respiratory treatment system for delivering pressurised air to an entrance to a patient's airways, the respiratory treatment system comprising at least;

a. a blower for generating the pressurised air;
b. a patient interface for sealing delivery of the pressurised air to the patient airways; and
c. the combined one way inspiratory valve and expiratory release valve assembly as described above, for controlling air flow to the patient interface.

In examples the system:
a) comprises a pressure sensor configured to measure pressure within the patient interface, wherein the system controls the blower based on data from the pressure sensor; b) reduces flow from the blower when the pressure sensor detects that the patient is exhaling; c) further comprises a conduit for delivering of the pressurised air to the patient interface, wherein the combined valve is included in the conduit or the patient interface; d) comprises a portable integrated blower/patent interface system wearable on the patient's face or head; and/or f) is arranged to be powered by one or more batteries.

Another aspect of the present technology comprises a method of characterising vent flow in a respiratory treatment system for delivering a pressurised air to an entrance to a patient's airways, the system comprising a combined one way inspiratory valve and an expiratory release valve as described above, the method comprising the steps of, for at least a first treatment pressure:

a) performing at least one simulated breathing cycle with the respiratory treatment system;
b) during the simulated breathing cycle procedure, measuring the flow rate through the valve vent, the pressure upstream of the valve and the pressure downstream of the valve;
c) plotting vent flow rate against a ratio of the pressures on both sides of the diaphragm;
d) identifying if there are any boundary points dividing the plotted data into one or more contiguous zones according to trends in the data;
e) deriving equations for best fit curves for the data in each of the identified zones; and
f) deriving, from the fitted equations, at least the coefficients and constants characterising the respective function between the pressure ratio and the vent flow for each zone, at least at the first treatment pressure.

In examples:
a) the ratio of the pressures is the ratio of the pressure upstream of the valve to the pressure downstream of the valve; b) the method further comprises repeating steps a) to e) for at least one second treatment pressure, to derive, from the respective fitted equations, the coefficients and constants characterising the respective function for each zone at the at least one second treatment pressure; c) the method further comprises interpolating the derived coefficients and constants to derive further coefficients and constants for treatment pressures other than the at least first and second treatment pressure; d) the method further comprises using the derived and/or the interpolated coefficients and constants to, for a given ratio of the pressures upstream and downstream of the valve, calculate a respective vent flow, for one or more respective treatment pressures; e) the method further comprises pre-calculating and tabulating the derived and the interpolated coefficients for a number of different treatment pressures, and using the tabulated numbers as a reference for less computationally demanding derivation of the vent flow at various pressures; f) the method further comprises using the derived vent flows to derive the patient flow at the respective treatment pressure; g) the method further comprises using the derived and/or tabulated vent flows to derive the patient flow at the respective treatment pressure; h) calculating the patient flow at the respective treatment pressure also includes measuring the flow rate of a blower of the respiratory treatment system and calculating an unintended leak at the patient interface; and/or i) the step of dividing the plotted data into a plurality of contiguous zones comprises dividing the plotted data into three contiguous zones.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

3 BRIEF DESCRIPTION OF THE DRAWINGS

The present technology is illustrated by way of example, and not by way of limitation, in the figures of the accompanying drawings, in which like reference numerals refer to similar elements including:

3.1 RESPIRATORY THERAPY SYSTEMS

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

3.2 RESPIRATORY SYSTEM AND FACIAL ANATOMY

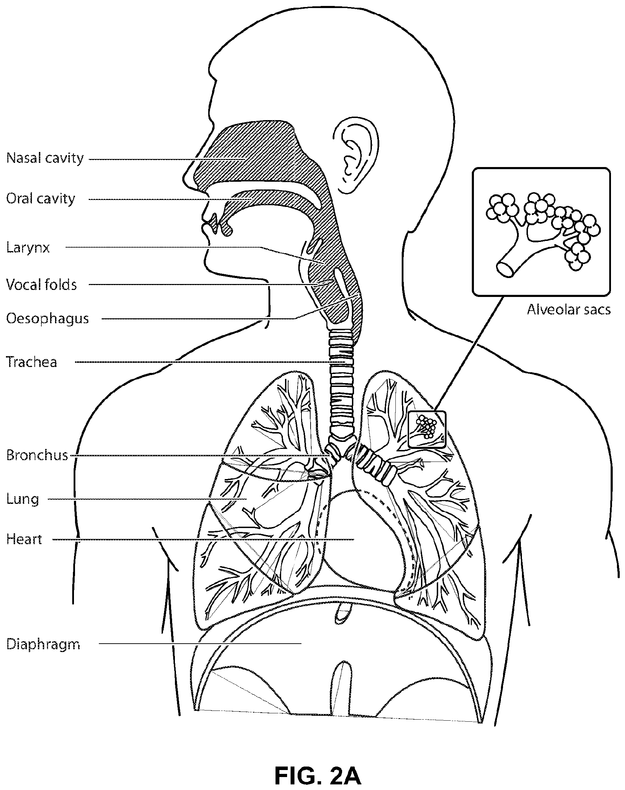

Fig. 2A shows an overview of a human respiratory system including the nasal and oral cavities, the larynx, vocal folds, oesophagus, trachea, bronchus, lung, alveolar sacs, heart and diaphragm.

3.3 PATIENT INTERFACE

Fig. 3A shows a patient interface in the form of a nasal mask in accordance with one form of the present technology.

3.4 RPT DEVICE

Fig. 4A shows an RPT device in accordance with one form of the present technology.

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

Fig. 4C is a schematic diagram of the electrical components of an RPT device in accordance with one form of the present technology.

Fig. 4D is a schematic diagram of the algorithms implemented in an RPT device in accordance with one form of the present technology.

Fig. 4E is a flow chart illustrating a method carried out by the therapy engine module of Fig. 4D in accordance with one form of the present technology.

3.5 HUMIDIFIER

Fig. 5A shows an isometric view of a humidifier in accordance with one form of the present technology.

Fig. 5B shows an isometric view of a humidifier in accordance with one form of the present technology, showing a humidifier reservoir 5110 removed from the humidifier reservoir dock 5130.

3.6 BREATHING WAVEFORMS

Fig. 6 shows a model typical breath waveform of a person while sleeping.

3.7 PATIENT INTERFACE, AIR CIRCUIT AND VALVE ASSEMBLY OF THE PRESENT TECHNOLOGY

Fig. 7 shows a system in accordance with one form of the present technology comprising a patient interface, an air circuit and an RPT device.

Fig. 8 shows a diagrammatic cross-section view of a valve assembly according to one form of the technology.

Fig. 9 shows a perspective view of a valve member in accordance with one form of the technology

Fig. 10 shows a diagrammatic cross section of the valve member of Fig. 10 with a duckbill valve in a closed configuration.

Fig. 11 shows a top view of a valve member in accordance with one form of the technology.

Fig. 12 shows a side view of the valve member of Fig. 11.

Fig. 13 shows a diagrammatic cross section of the valve member of Fig. 10 with a duckbill valve in an open configuration.

Fig. 14 shows an exploded cross-section view of the valve assembly of Fig. 8.

Fig. 15 shows a cross-section view of the valve assembly of Fig. 8 with the upstream housing portion separated from the downstream housing portion.

Fig. 16 shows a diagrammatic cross-section view of a valve assembly according to one form of the technology, with the duckbill valve open and the diaphragm blocking flow to the vent.

Fig. 17 shows an enlarged view of the diaphragm and vent of the valve assembly of Fig. 16.

Fig. 18 shows a diagrammatic cross-section view of the valve assembly of Fig. 8 with the duckbill valve closed and the diaphragm allowing flow to the vent.

Fig. 19 shows an enlarged view of the diaphragm and vent of the valve assembly of Fig. 18.

Fig. 20 shows a diagrammatic cross-section view of a valve assembly of according to one form of the technology, with the diaphragm allowing flow to the vent and the duckbill valve allowing a small flow.

Fig. 21 shows an enlarged diagrammatic cross-section view of a valve assembly according to one form of the technology, with the membrane shown in both the venting (dashed lines) and a non-venting configuration.

Fig. 22 shows a simplified breathing curve relating to the use of a valve member of the present technology.

Fig. 23 shows simplified vent flow curves relating to the use of a valve member of the present technology.

Fig. 24 shows an open duckbill valve according to one form of the technology, and a simulated flow through the duckbill valve.

Fig. 25 shows examples of an alternative form of valve member, which are not provided with duckbill valves, which have exemplary portions of different thickness.

Fig. 26 is a flow diagram and a formula related to the characteristics of a system for treating a respiratory disorder.

Fig. 27 shows part of an experimental setup for performing simulated breathing cycles.

Fig. 28 is a plot of vent flow rate vs pressure ratio for one form of the technology at one therapy pressure setting.

Fig. 29 is a graph showing estimated and measured patient flow rates vs time for a patient interface of one form of the present technology at a therapy pressure of 6 cmH2O.

Fig. 30 is a graph showing estimated and measured patient flow rates vs time for a patient interface of one form of the present technology at a therapy pressure of 8 cmH2O.

Fig. 31 is a graph showing estimated and measured patient flow rates vs time for a patient interface of one form of the present technology at a therapy pressure of 12 cmH2O.

Fig. 32 is a perspective view of a passive humidifier according to one form of the technology.

Fig 33 is an exploded view of the passive humidifier of Fig. 32.

Fig. 34 shows a schematic cross-section view of a valve assembly according to another form of the technology.

Fig. 35 shows a top perspective view of a downstream housing portion of the valve assembly of Fig. 34.

Fig. 36 shows a top perspective view of a downstream housing portion of the alternative valve assembly of Fig. 8.

Fig. 37 shows a perspective view of a valve member in accordance with one form of the technology.

Fig. 38 shows a perspective view of a valve member in accordance with one form of the technology.

Fig. 39 shows a perspective view of a valve member in accordance with one form of the technology.

Fig. 40 shows a side perspective view of a valve member in accordance with one form of the technology.

Fig. 41 is a flow chart showing steps in a method of characterising a vent flow in a respiratory treatment system according to one form of the technology.

## 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.
The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 4.1 THERAPY

In one form, the present technology comprises a method for treating a respiratory disorder comprising applying positive pressure to the entrance of the airways of a patient 1000.
In certain examples of the present technology, a supply of air at positive pressure is provided to the nasal passages of the patient via one or both nares.
In certain examples of the present technology, mouth breathing is limited, restricted or prevented.

### 4.2 RESPIRATORY THERAPY SYSTEMS

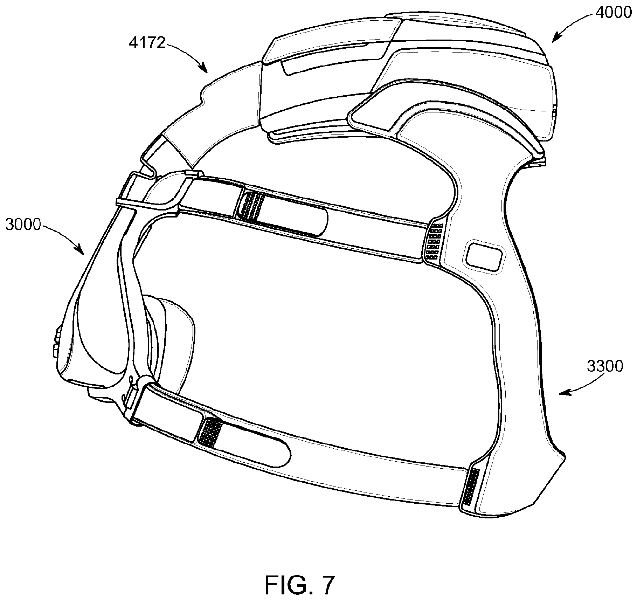

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.
One form of such a system is shown in Fig. 7. The example shown in Fig. 7 is a portable integrated blower/patent interface system wearable on the patient's face or head. The example shown in Fig. 7 may be battery powered by one or more batteries.

### 4.3 PATIENT INTERFACE

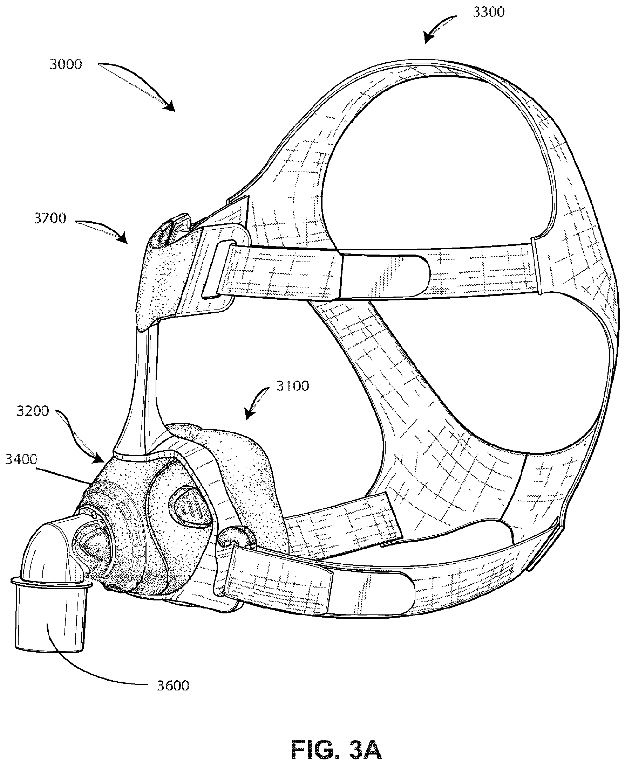

A non-invasive patient interface 3000, such as that shown in Fig. 3A, in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

4.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH2O, or at least 10cmH2O, or at least 20 cmH2O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 4.4.1 RPT device mechanical & pneumatic components

An RPT device may comprise one or more of the following components in an integral unit. In an alternative form, one or more of the following components may be located as respective separate units.

### 4.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 4.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 4.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH2O to about 20 cmH2O, or in other forms up to about 30 cmH2O when delivering respiratory pressure therapy. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

The pressure generator 4140 may be under the control of the therapy device controller 4240.

In other forms, a pressure generator 4140 may be a piston-driven pump, a pressure regulator connected to a high pressure source (e.g. compressed air reservoir), or a bellows.

### 1.1.1.1 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000.

In one form, a signal from a transducer 4270 may be filtered, such as by low-pass, high-pass or band-pass filtering.

#### *4.4.1.4.1 Flow rate sensor*

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

#### *4.4.1.4.2 Pressure sensor*

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 and representing a pressure is received by the central controller 4230.

#### *4.4.1.4.3 Motor speed transducer*

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 4.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 4.4.2 RPT device electrical components

### 4.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 4.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

### 4.4.2.3 Central controller

In one form of the present technology, the central controller 4230 is one or a plurality of processors suitable to control an RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 which may be implemented with processor-control instructions, expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

### 4.4.2.4 Clock

The RPT device 4000 may include a clock 4232 that is connected to the central controller 4230.

### 4.4.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

### 4.4.2.6 Protection circuits

The one or more protection circuits 4250 in accordance with the present technology may comprise an electrical protection circuit, a temperature and/or pressure safety circuit.

### 4.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally, or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 4.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 4.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

#### 4.4.2.9.1 Display driver

A display driver 4292 receives as an input the characters, symbols, or images intended for display on the display 4294, and converts them to commands that cause the display 4294 to display those characters, symbols, or images.

#### 4.4.2.9.2 Display

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 4.4.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs, such as with processor control instructions to be executed by one or more processor(s), stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 4.4.3.1 Pre-processing module

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface pressure *Pm,* the vent flow rate *Qv,* the respiratory flow rate *Qr,* and the leak flow rate *Ql.*

In various forms of the present technology, the pre-processing module 4310 comprises one or more of the following algorithms: interface pressure estimation 4312, vent flow rate estimation 4314, leak flow rate estimation 4316, and respiratory flow rate estimation 4318.

### 4.4.3.1.1 Interface pressure estimation

In one form of the present technology, an interface pressure estimation algorithm 4312 receives as inputs a signal from the pressure sensor 4272 indicative of the pressure in the pneumatic path proximal to an outlet of the pneumatic block (the device pressure *Pd*) and a signal from the flow rate sensor 4274 representative of the flow rate of the airflow leaving the RPT device 4000 (the device flow rate *Qd*). The device flow rate *Qd,* absent any supplementary gas 4180, may be used as the total flow rate *Qt*. The interface pressure algorithm 4312 estimates the pressure drop *ΔP* through the air circuit 4170. The dependence of the pressure drop *ΔP* on the total flow rate *Qt* may be modelled for the particular air circuit 4170 by a pressure drop characteristic *ΔP(Q)*. The interface pressure estimation algorithm, 4312 then provides as an output an estimated pressure, *Pm,* in the patient interface 3000. The pressure, *Pm,* in the patient interface 3000 may be estimated as the device pressure *Pd* minus the air circuit pressure drop *ΔP*.

### 4.4.3.1.2 Vent flow rate estimation

In one form of the present technology, a vent flow rate estimation algorithm 4314 receives as an input an estimated pressure, *Pm,* in the patient interface 3000 from the interface pressure estimation algorithm 4312 and estimates a vent flow rate of air, *Qv,* from a vent 3400 in a patient interface 3000. The dependence of the vent flow rate *Qv* on the interface pressure *Pm* for the particular vent 3400 in use may be modelled by a vent characteristic *Qv(Pm)*.

In another form of the technology the vent flow *Qv* is estimated based on one or more ratios between the pressures on both sides of the diaphragm 6060 of a valve (namely the pressure at the blower pressure side (Bp) and the pressure at the patient interface side (Pp)), as is described further below.

### 4.4.3.1.3 Leak flow rate estimation

In one form of the present technology, a leak flow rate estimation algorithm 4316 receives as an input a total flow rate, *Qt,* and a vent flow rate *Qv,* and provides as an output an estimate of the leak flow rate *Ql*. In one form, the leak flow rate estimation algorithm estimates the leak flow rate *Ql* by calculating an average of the difference between total flow rate *Qt* and vent flow rate *Qv* over a period sufficiently long to include several breathing cycles, e.g. about 10 seconds.

In one form, the leak flow rate estimation algorithm 4316 receives as an input a total flow rate *Qt,* a vent flow rate *Qv,* and an estimated pressure, *Pm,* in the patient interface 3000, and provides as an output a leak flow rate *Ql,* by calculating a leak conductance, and determining a leak flow rate *Ql* to be a function of leak conductance and pressure, *Pm.* Leak conductance is calculated as the quotient of low pass filtered non-vent flow rate equal to the difference between total flow rate *Qt* and vent flow rate *Qv,* and low pass filtered square root of pressure *Pm,* where the low pass filter time constant has a value sufficiently long to include several breathing cycles, e.g. about 10 seconds. The leak flow rate *Ql* may be estimated as the product of leak conductance and a function of pressure, *Pm.*

### 4.4.3.1.4 Respiratory flow rate estimation

In one form of the present technology, a respiratory flow rate estimation algorithm 4318 receives as an input a total flow rate, *Qt,* a vent flow rate, *Qv,* and a leak flow rate, *Ql,* and estimates a respiratory flow rate of air, *Qr,* to the patient, by subtracting the vent flow rate *Qv* and the leak flow rate *Ql* from the total flow rate *Qt.*

### 4.4.3.2 Therapy Engine Module

In one form of the present technology, a therapy engine module 4320 receives as inputs one or more of a pressure, *Pm,* in a patient interface 3000, and a respiratory flow rate of air to a patient, *Qr,* and provides as an output one or more therapy parameters.

In one form of the present technology, a therapy parameter is a treatment pressure *Pt.*

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

### 4.4.3.2.1 Phase determination

In one form of the present technology, the RPT device 4000 does not determine phase.

In one form of the present technology, a phase determination algorithm 4321 receives as an input a signal indicative of respiratory flow rate, $Qr,$ and provides as an output a phase $\Phi$ of a current breathing cycle of a patient 1000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate $Qr$ has a value that exceeds a positive threshold, and a discrete value of 0.5 revolutions (thereby "cycling" the RPT device 4000) when a respiratory flow rate $Qr$ has a value that is more negative than a negative threshold. The inhalation time $Ti$ and the exhalation time $Te$ may be estimated as typical values over many respiratory cycles of the time spent with phase $\Phi$ equal to 0 (indicating inspiration) and 0.5 (indicating expiration) respectively.

Another implementation of discrete phase determination provides a trivalued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate $Qr.$ A continuous value of phase determined in this implementation is often referred to as "fuzzy phase". In one implementation of a fuzzy phase determination algorithm 4321, the following rules are applied to the respiratory flow rate $Qr$:

1. If $Qr$ is zero and increasing fast then $\Phi$ is 0 revolutions.

2. If $Qr$ is large positive and steady then $\Phi$ is 0.25 revolutions.

3. If $Qr$ is zero and falling fast, then $\Phi$ is 0.5 revolutions.

4. If $Qr$ is large negative and steady then $\Phi$ is 0.75 revolutions.

5. If $Qr$ is zero and steady and the 5-second low-pass filtered absolute value of $Qr$ is large then $\Phi$ is 0.9 revolutions.

6. If $Qr$ is positive and the phase is expiratory, then $\Phi$ is 0 revolutions.

7. If $Qr$ is negative and the phase is inspiratory, then $\Phi$ is 0.5 revolutions.

8. If the 5-second low-pass filtered absolute value of $Qr$ is large, $\Phi$ is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase $\Phi$ is first discretely estimated from the respiratory flow rate $Qr$ as described above, as are the inhalation time $Ti$ and the exhalation time $Te$. The continuous phase $\Phi$ at any instant may be determined as the half the proportion of the inhalation time $Ti$ that has elapsed since the previous trigger instant, or 0.5 revolutions plus half the proportion of the exhalation time *Te* that has elapsed since the previous cycle instant (whichever instant was more recent).

### 4.4.3.2.2 Waveform determination

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure *Pt* that varies as a function of phase $\Phi$ of a respiratory cycle of a patient according to a waveform template $\Pi(\Phi)$.

In one form of the present technology, a waveform determination algorithm 4322 provides a waveform template $\Pi$($\Phi$) with values in the range [0, 1] on the domain of phase values $\Phi$ provided by the phase determination algorithm 4321 to be used by the therapy parameter determination algorithm 4329.

In one form, suitable for either discrete or continuously-valued phase, the waveform template $\Pi(\Phi)$ is a square-wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template $\Pi(\Phi)$ is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template $\Pi(\Phi)$ from a library of waveform templates, dependent on a setting of the RPT device. Each waveform template $\Pi$($\Phi$) in the library may be provided as a lookup table of values $\Pi$ against phase values $\Phi$. In other forms, the waveform determination algorithm 4322 computes a waveform template $\Pi(\Phi)$ "on the fly" using a predetermined functional form, possibly parametrised by one or more parameters (e.g. time constant of an exponentially curved portion). The parameters of the functional form may be predetermined or dependent on a current state of the patient 1000.

In some forms of the present technology, suitable for discrete bi-valued phase of either inhalation ($\Phi$ = 0 revolutions) or exhalation ($\Phi$ = 0.5 revolutions), the waveform determination algorithm 4322 computes a waveform template $\Pi$ "on the fly" as a function of both discrete phase $\Phi$ and time t measured since the most recent trigger instant. In one such form, the waveform determination algorithm 4322 computes the waveform template $\Pi(\Phi, t)$ in two portions (inspiratory and expiratory) as follows:

$$\Pi\left(\Phi,t\right)=\begin{cases} \Pi_i\left(t\right), & \Phi=0 \\ \Pi_e\left(t-T_i\right), & \Phi=0.5 \end{cases}$$

where $\Pi_i(t)$ and $\Pi_e(t)$ are inspiratory and expiratory portions of the waveform template $\Pi(\Phi, t)$. In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

### 4.4.3.2.3 Ventilation determination

In one form of the present technology, a ventilation determination algorithm 4323 receives an input a respiratory flow rate *Qr,* and determines a measure indicative of current patient ventilation, *Vent.*

In some implementations, the ventilation determination algorithm 4323 determines a measure of ventilation *Vent* that is an estimate of actual patient ventilation. One such implementation is to take half the absolute value of respiratory flow rate, *Qr,* optionally filtered by low-pass filter such as a second order Bessel low-pass filter with a corner frequency of 0.11 Hz.

In other implementations, the ventilation determination algorithm 4323 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate *Qr* produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle K proportion (by time) of the inspiratory portion, where $0 < K < 1$. There is an arbitrarily large number of measures that are exactly proportional to ventilation if the flow rate shape is constant.

### 4.4.3.2.4 Determination of Inspiratory Flow Limitation

In one form of the present technology, the central controller 4230 executes an inspiratory flow limitation determination algorithm 4324 for the determination of the extent of inspiratory flow limitation.

In one form, the inspiratory flow limitation determination algorithm 4324 receives as an input a respiratory flow rate signal $Qr$ and provides as an output a metric of the extent to which the inspiratory portion of the breath exhibits inspiratory flow limitation.

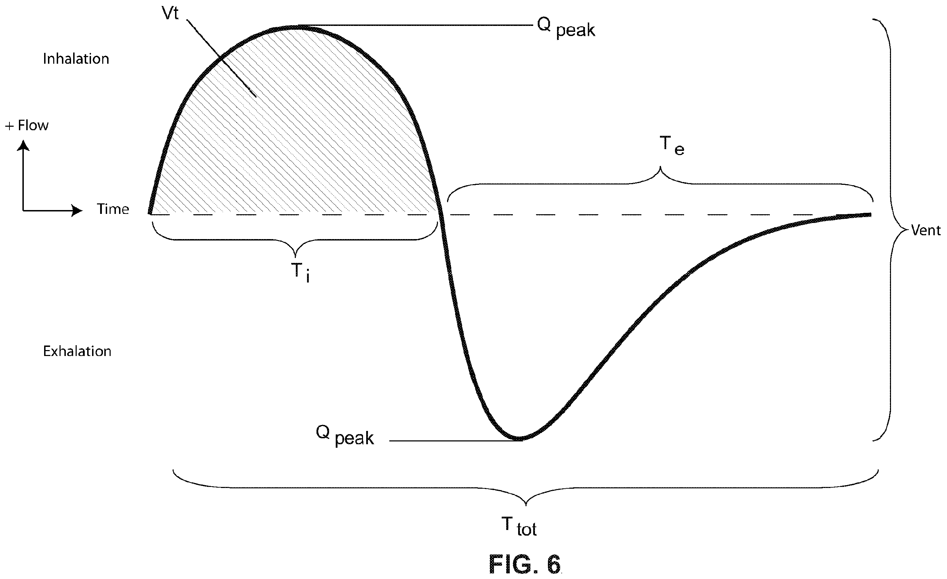

In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath, similar to the inspiratory portion of the breath shown in Fig. 6A. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the central controller 4230 for the preceding several inspiratory events. This is repeated over the same inspiratory events for the second such point, and so on. Thus, for example, sixty-five scaled data points are generated by the central controller 4230, and represent a moving average of the preceding several inspiratory events, e.g., three events. The moving average of continuously updated values of the (e.g., sixty-five) points are hereinafter called the "scaled flow rate ", designated as Qs(t). Alternatively, a single inspiratory event can be utilised rather than a moving average.

From the scaled flow rate, two shape factors relating to the determination of partial obstruction may be calculated.

Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.

Shape factor 2 is calculated as the RMS deviation from unit scaled flow rate, taken over the middle (e.g. thirty-two) points. An RMS deviation of about 0.2 units is taken to be normal. An RMS deviation of zero is taken to be a totally flow-limited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can be other than those described.

### 4.4.3.2.5 Determination of apneas and hypopneas

In one form of the present technology, the central controller 4230 executes an apnea / hypopnea determination algorithm 4325 for the determination of the presence of apneas and/or hypopneas.

In one form, the apnea / hypopnea determination algorithm 4325 receives as an input a respiratory flow rate signal $Qr$ and provides as an output a flag that indicates that an apnea or a hypopnea has been detected.

In one form, an apnea will be said to have been detected when a function of respiratory flow rate $Qr$ falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate $Qr$ falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

### 4.4.3.2.6 Determination of snore

In one form of the present technology, the central controller 4230 executes one or more snore determination algorithms 4326 for the determination of the extent of snore.

In one form, the snore determination algorithm 4326 receives as an input a respiratory flow rate signal $Qr$ and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal *Qr* to reduce background noise, e.g., the sound of airflow in the system from the blower.

### 4.4.3.2.7 Determination of airway patency

In one form of the present technology, the central controller 4230 executes one or more airway patency determination algorithms 4327 for the determination of the extent of airway patency.

In one form, the airway patency determination algorithm 4327 receives as an input a respiratory flow rate signal *Qr,* and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure *Pt.* In one implementation, the forced oscillation is of frequency 2 Hz with amplitude about 1 cmH$_2$O.

In one form, airway patency determination algorithm 4327 receives as an input a respiratory flow rate signal *Qr,* and determines the presence or absence of a cardiogenic signal. The absence of a cardiogenic signal is taken to be an indication of a closed airway.

### 4.4.3.2.8 Determination of target ventilation

In one form of the present technology, the central controller 4230 takes as input the measure of current ventilation, *Vent,* and executes one or more target ventilation determination algorithms 4328 for the determination of a target value *Vtgt* for the measure of ventilation.

In some forms of the present technology, there is no target ventilation determination algorithm 4328, and the target value *Vtgt* is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value *Vtgt* from a value *Vtyp* indicative of the typical recent ventilation of the patient.

In some forms of adaptive servo-ventilation, the target ventilation *Vtgt* is computed as a high proportion of, but less than, the typical recent ventilation *Vtyp.* The high proportion in such forms may be in the range (80%, 100%), or (85%, 95%), or (87%, 92%).

In other forms of adaptive servo-ventilation, the target ventilation *Vtgt* is computed as a slightly greater than unity multiple of the typical recent ventilation *Vtyp.*

The typical recent ventilation *Vtyp* is the value around which the distribution of the measure of current ventilation *Vent* over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation *Vtyp* from the measure of current ventilation, *Vent.* One such measure is the output of a low-pass filter on the measure of current ventilation *Vent,* with time constant equal to one hundred seconds.

### 4.4.3.2.9 Determination of therapy parameters

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure *Pt.* In one implementation of this form, the therapy parameter determination algorithm 4329 determines the treatment pressure *Pt* using the equation

$$Pt = A\Pi\left(\Phi, t\right) + P_0 \tag{1}$$

where:

- A is the amplitude,
- $\Pi(\Phi, t)$ is the waveform template value (in the range 0 to 1) at the current value $\Phi$ of phase and $t$ of time, and
- $P_0$ is a base pressure.

If the waveform determination algorithm 4322 provides the waveform template $\Pi(\Phi, t)$ as a lookup table of values $\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude A and the base pressure $P_0$ may be set by the therapy parameter determination algorithm 4329 depending on the chosen respiratory pressure therapy mode in the manner described below.

### 4.4.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of air whose interface pressure *Pm* at the patient interface 3000 is equal to the treatment pressure *Pt.*

### 4.4.3.4 Detection of fault conditions

In one form of the present technology, the central controller 4230 executes one or more methods 4340 for the detection of fault conditions. The fault conditions detected by the one or more methods 4340 may include at least one of the following:

- Power failure (no power, or insufficient power)
- Transducer fault detection
- Failure to detect the presence of a component
- Operating parameters outside recommended ranges (e.g. pressure, flow rate, temperature, PaO2)
- Failure of a test alarm to generate a detectable alarm signal.

Upon detection of the fault condition, the corresponding algorithm 4340 signals the presence of the fault by one or more of the following:

- Initiation of an audible, visual &/or kinetic (e.g. vibrating) alarm
- Sending a message to an external device
- Logging of the incident

### 4.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349.

### 4.5.1 Valve assembly

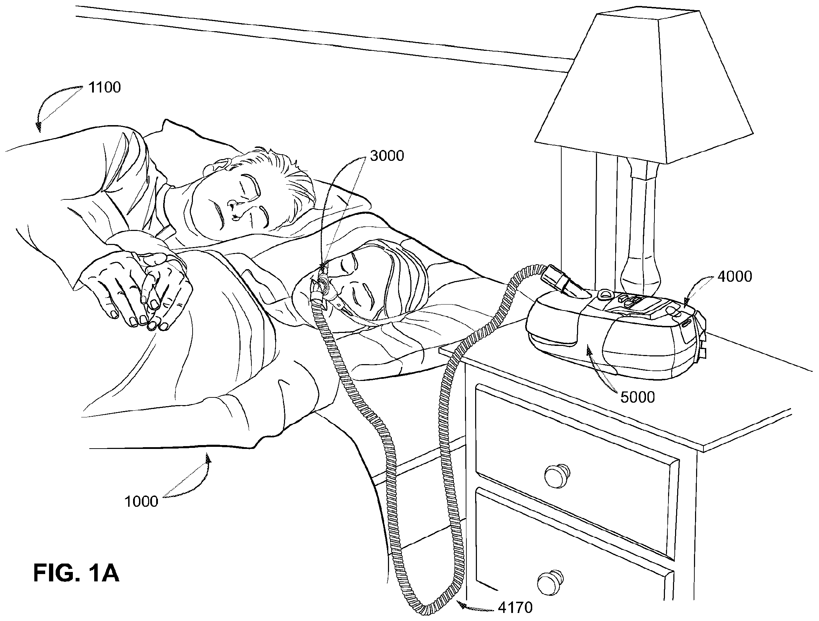

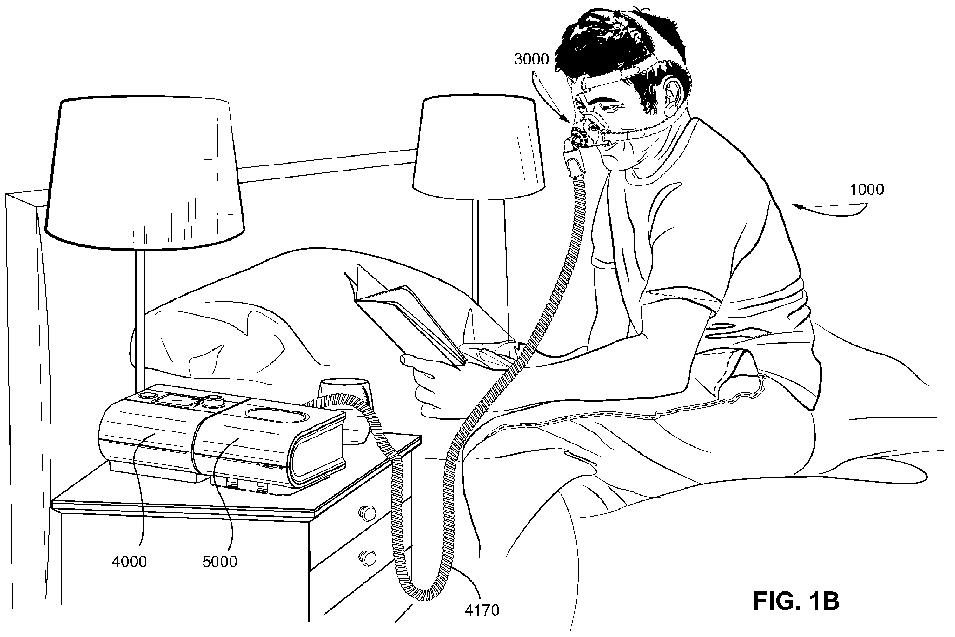

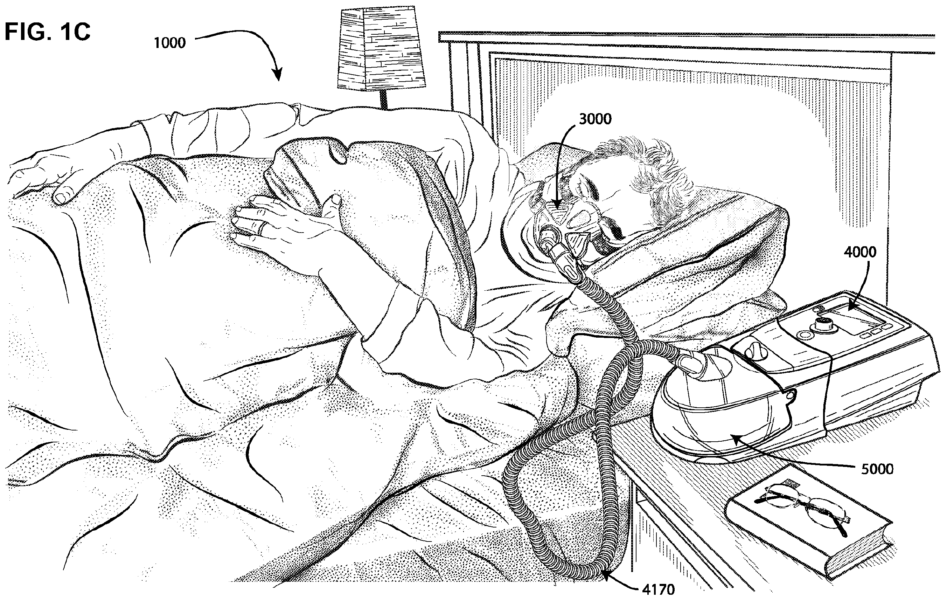

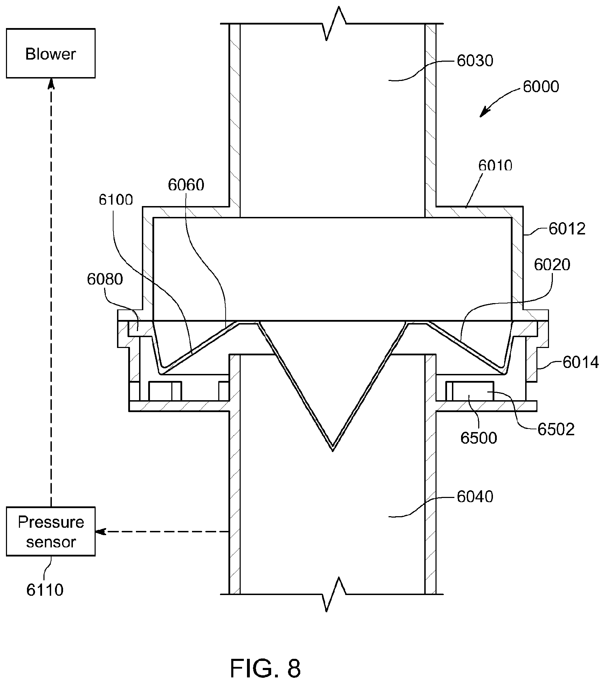

In one form of the present technology, the air circuit 4170 (Fig. 1A to 1C) or the patient interface 3000 (Fig. 1A to 1C) of a positive air-pressure (PAP) therapy system can comprise a valve assembly 6000 (Fig. 8). The valve assembly 6000 may be configured as a combined one way inspiratory valve and expiratory release valve assembly.

The valve assembly 6000 should generally be located as close to the patient interface 3000 as possible. If the valve assembly 6000 is too far away from the patient interface 3000, this will create a larger dead space and may lead to an unacceptable level of $CO_2$ accumulating in the mask space. Thus, the valve assembly 6000 may be located in or on patient interface 3000, or in/on the air circuit 4170, but close to the patient interface 3000. The valve assembly 6000 may comprise a housing 6010, and a pressure-activated valve member 6020.

The valve assembly 6000 may comprise:

- An inlet 6030 (also referred to as an RPT port);•

- An outlet 6040 (which in some embodiments may be located on the patient interface and is for that reason also referred to as a patient interface port); and

- A vent 6050 (also referred to as an ambient port).

In one form, the pressure-activated valve member 6020 has a dual function in which it combines a one-way inspiration or inspiratory valve, which can provide the airflow that pressurises the patient airways (only, or primarily, during inspiration), with an expiration release valve, which controls vent flow to the ambient atmosphere (only, or primarily, during expiration). Thus, the combined valve effectively controls both the airflow from the RPT device 4000 to the patient side of the mask 3000, as well as the airflow from the inside of the mask 3000 to the ambient atmosphere. In particular, the combined valve 6000 can be constructed and arranged to operate in two main states:

  i. A first state, in which air passes between the RPT port 6030 and the patient interface port 6040. whilst the flow path to ambient (e.g. the vent 6050) is closed; and

  ii. A second state, in which air passes from the patient interface port 6040 to the ambient port 6050, and from there to ambient, whilst the RPT port is closed (e.g. flow to the RPT port 6030 is blocked).

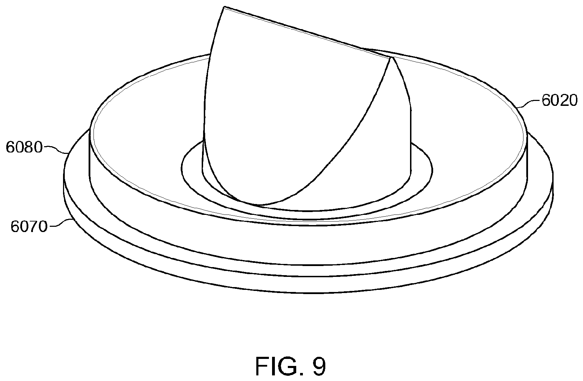

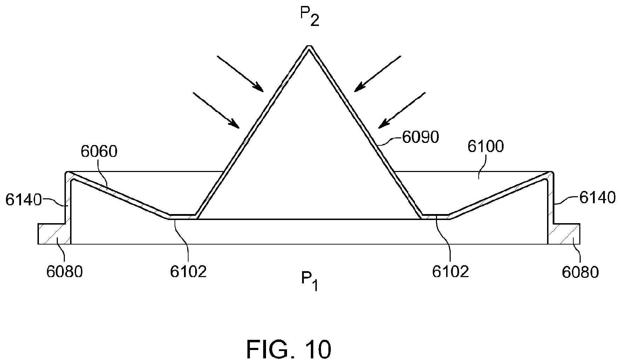

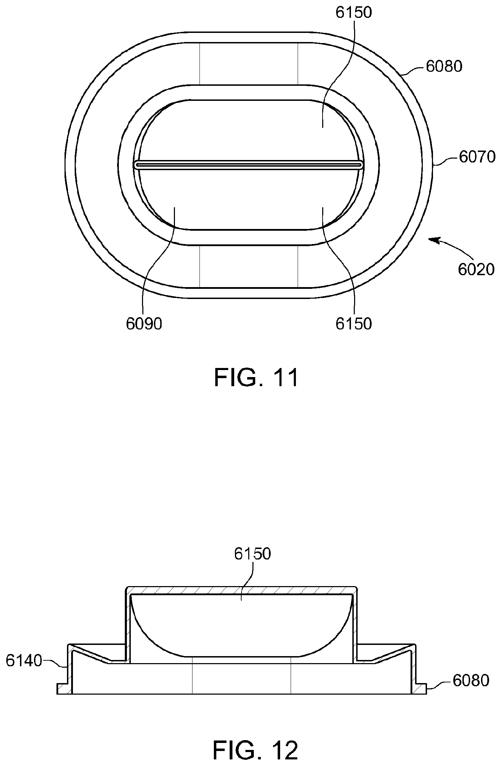

In one form, the valve assembly 6000 comprises a valve member, e.g. a diaphragm 6060, made from a silicone material and having a substantially circular outline e.g. as shown in Figs. 9 and 10. In other examples, the diaphragm 6060 may have an elongate form, e.g. an elliptical, oval or stadium shape outline, for example as shown in Figs. 11 and 12. In some examples, the cross-sectional shape of the patient interface port 6040 (or at least the inlet to that port) can be substantially the same as the shape of the diaphragm 6060, e.g. in examples with a substantially circular diaphragm 6060 the inlet to the patient interface port 6040 may be substantially circular; in examples with a substantially elliptical diaphragm 6060, the inlet to the patient interface port 6040 may be substantially elliptical.

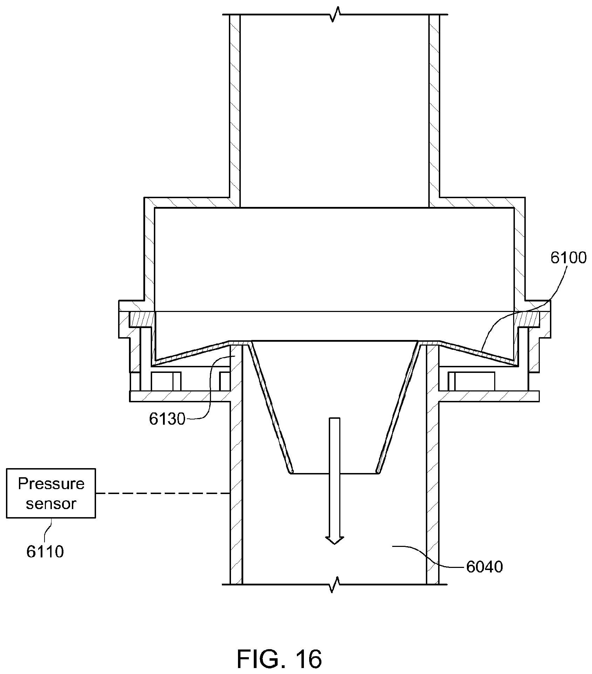

Referring next to Figs. 11 and 12, on the outer circumference 6070 of the diaphragm 6060 is a retention flange feature 6080 configured to locate and retain the diaphragm 6060 in the housing 6010. At the centre of the diaphragm 6060 there is a one way valve 6090 configured as a duckbill valve. With reference to Fig. 16, the valve 6090 allows air to pass through the diaphragm 6060 in one direction only, from the RPT port 6030 to the patient interface port 6040.

With reference to Fig. 10, the diaphragm 6060 has a thin membrane portion 6100 between the retention flange 6080 and the duckbill valve 6090. The retention flange 6080 may be the only part of the diaphragm 6060 to be fixed on assembly, allowing the membrane portion 6100 to move in response to the pressure differential acting on either side. Although they may be formed as one part, the duckbill valve 6090 and membrane portion 6100 may work independently to each other. The duckbill valve 6090 controls airflow from the RPT device 4000 (or blower, see inlet 6030 in Fig. 8) to the patient interface 3000 (or mask, see outlet 6040 in Fig. 8), and the membrane portion 6100 controls the airflow from the patient interface 3000 (or mask) through the vent 6050. The diaphragm 6060 is a dynamic component, reacting to any appreciable pressure change, including the breaths the user takes.

Exemplary characteristics of the diaphragm 6060 may include:

Material Preference: Silicone;

Shore Hardness: 30-60 Shore (A); and

Manufacturing Process: LSR or CMSR.

### 4.5.1.1 Duckbill valve

In one form, the diaphragm 6060 has a duckbill valve 6090 in the centre of the membrane portion 6100. The duckbill is a one-way valve allowing air to pass through the diaphragm 6060 in one direction only. The difference in pressure on both sides of the duckbill valve 6090 will determine if the valve is open or closed. The material, the size and shape of the duckbill valve 6090 will have an effect on the flow rate and impedance through the membrane.

Figs 9-12 shows examples of the duckbill valve 6090 in a closed configuration.

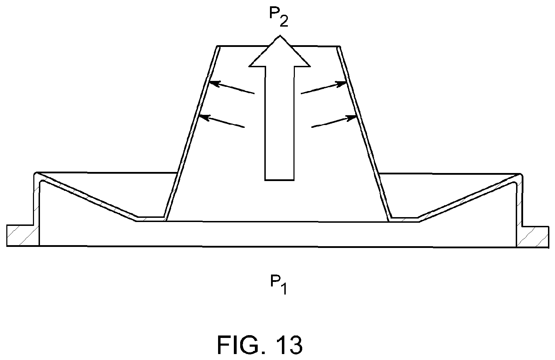

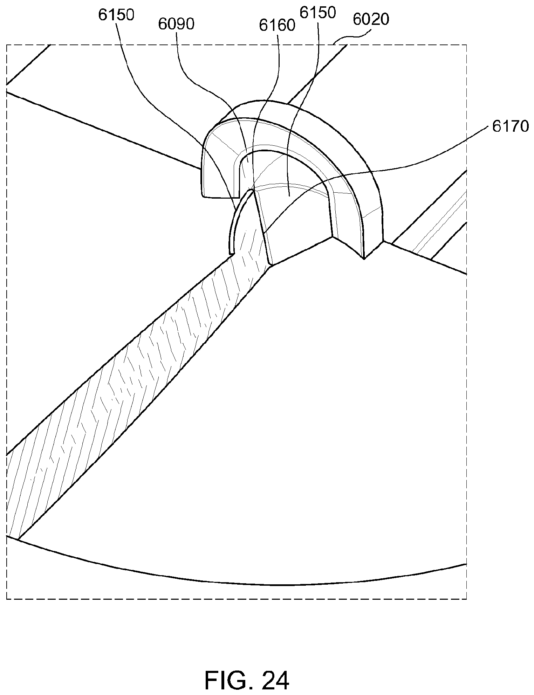

Figs. 13 and 24 show example duckbill valves 6090 in an open configuration.

### 4.5.1.2 Valve assembly

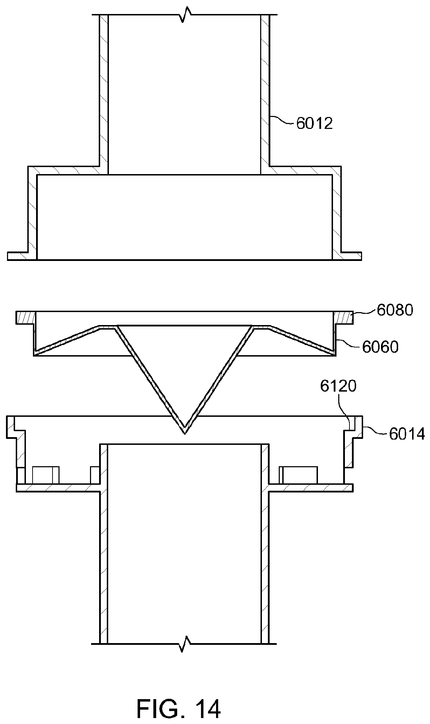

Referring next to Figs. 8, 14 and 15, the retention flange 6080 locates the diaphragm 6060 between two housing portions 6012, 6014. The retention flange 6080 may also operate as a seal between the upstream and downstream portions 6012, 6014 of the housing 6010, e.g. between the RPT device (or blower) and mask air paths, and between the RPT device and ambient port. When integrated, the retention flange 6080 may be the only part of the diaphragm 6060 to be fixed on assembly, therefore allowing the membrane portion 6100 to move in response to differences in pressure on the upstream and downstream sides.

In one form, the present technology comprises a pressure sensor 6110 connected, directly or indirectly (i.e. by being connected to conduit 4170), to the patient interface 3000 (see Fig. 16). A controller (for example the therapy device controller 4240) monitors the pressure in the patient interface 3000 (measured by the pressure sensor 6110) and controls the pressure generator 4140 to adjust the flow rate of the generated air supply.

The retention flange 6080 of the diaphragm 6060 can sit within a recess 6120 provided to one housing portion, e.g. a first plastic component. This feature may ensure the diaphragm 6060 is correctly located within the housing 6010 and stop any unwanted lateral movement.

When assembled, the retention flange 6080 of the diaphragm 6060 can create an interference seal between the two housing portions 6012, 6014, as seen in Fig. 8.

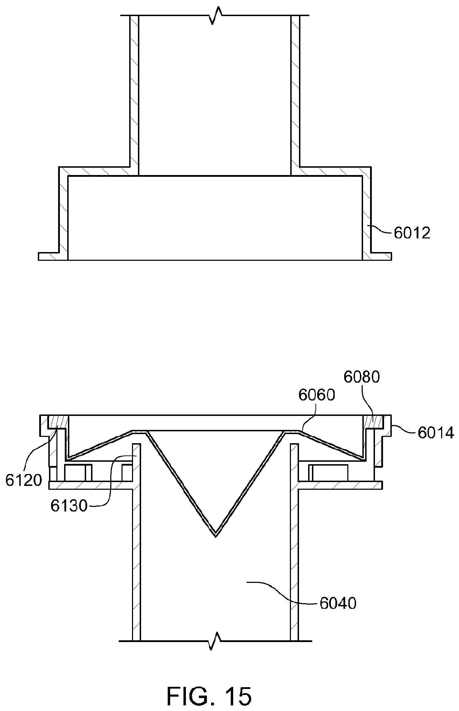

Referring next to Figs. 15-17, the intermediate portion 6102 of the diaphragm membrane 6100, which is somewhat more outer and/or peripheral than the inner duckbill valve portion 6090, effectively controls the access to the vent from the patient interface port 6040 by interacting (e.g. substantially vertically in the upstream-downstream direction) with an annular rib 6130 (which could extend inwardly - e.g. in the upstream direction). The contact between the portion 6102 of the diaphragm membrane 6100 and the annular rib 6130 controls an outlet flow path from the downstream (on the mask side) space of the housing to ambient.

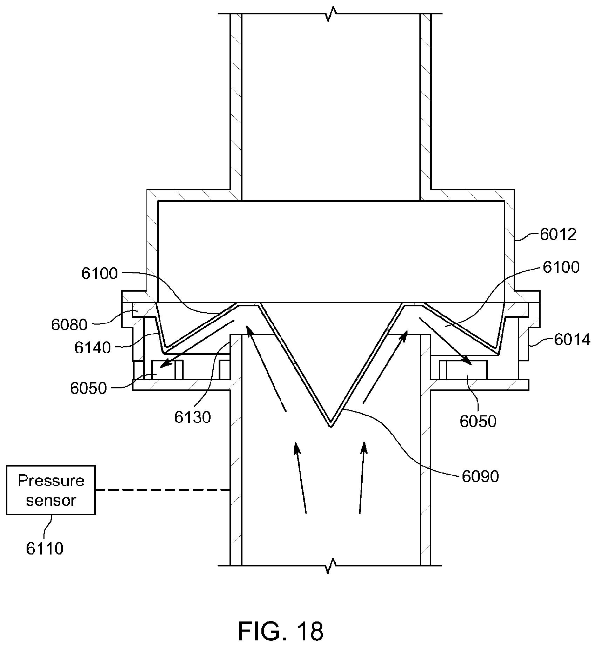

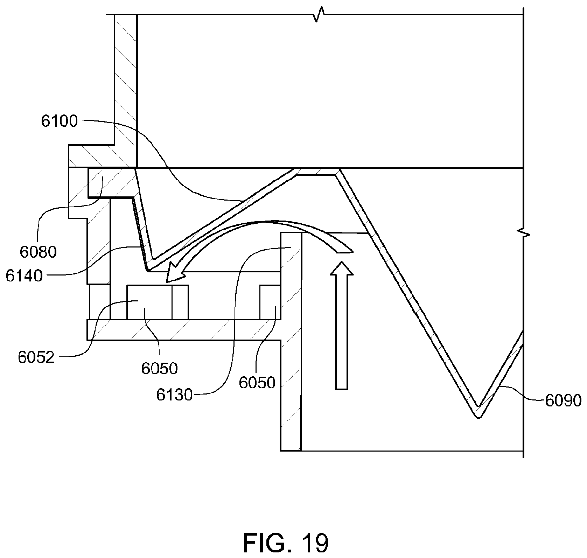

Referring next to Figs. 18 and 19, the clearance gap between the diaphragm membrane 6100 and the rib 6130 (outlet flow path inlet) defines part of a flow path from the patient interface 3000 to the vent 6050 and thereby controls the vent flow. In some arrangements the vent 6050 comprises one vent opening, in others a plurality of vent openings 6052. The vent opening(s) 6052 can have a larger and more open combined area than the area defined by the maximum expected clearance gap between the diaphragm membrane 6100 and the rib 6130. The outer vent openings 6052 may therefore have less effect on the flow rate through the vent 6050 than the size of the clearance gap. In examples, the flow rate through the vent is, at all times, solely a function of the size of clearance gap between the diaphragm membrane 6100 and the rib 6130.

### 4.5.1.3 Valve assembly - inspiration

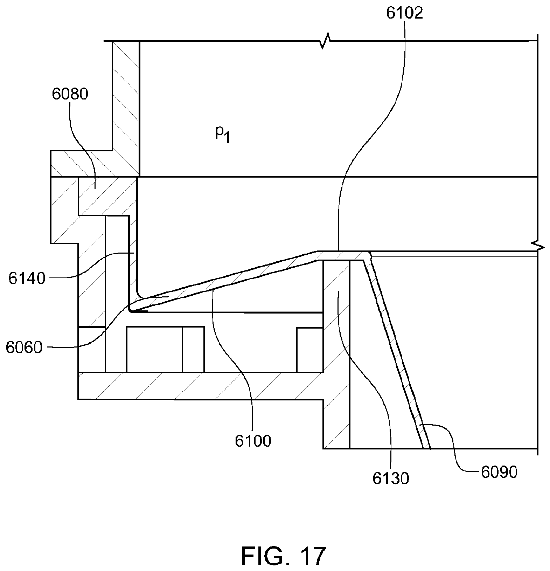

Referring next to Fig. 16 and 17, in certain forms of the technology, on inspiration the patient interface pressure drops due to the increase of volume created by the user's diaphragm contracting (moving down). The pressure sensor 6110 may record this change and signal the RPT device to provide more airflow to achieve a predetermined pressure and/or maintain a set pressure. The RPT device airflow causes an increase in pressure (P1) on the RPT device side of the valve assembly 6000. This pressure (P1) is greater than the patient interface pressure (P2) and causes the diaphragm membrane 6100 to move towards the patient interface port rib 6130, until the resistance force of the membrane (caused by the resilient nature of the membrane and defined by the membrane's structural configuration and resilient properties of the membrane material) is overcome and contact is made with the patient interface port rib 6130. This causes the expiratory valve to be at least partially shut down, reducing or preventing the flow through the valve vent to the ambient atmosphere. A specific membrane design and/or material may cause the valve to close at a specific targeted (predetermined) pressure differential. If the resistance force of the membrane is negligible, it may be stated that the valve closes as soon as the upstream pressure is higher than the downstream one. As will be explained in more detail later, the pressure differential may also cause the duckbill valve 6090 to open, allowing air flow into the patient interface 3000. This airflow will cause the patient interface pressure (P2) to increase until the desired therapy pressure is reached.

The fact that the diaphragm membrane 6100 stays in this closed (or at least partially closed) position during inspiration reduces both the air flow through the vent 6050 and the potential associated pressure drop. As a result, the RPT device does not have to work as hard, in order to maintain the required mask pressure P2. Thus, such a reduction in the airflow/pressure loss during inspiration improves the overall efficiency of the blower and the RPT device.

#### 4.5.1.3.1 Duckbill valve - inspiration

As mentioned previously in the text, on inspiration the RPT device pressure is greater than the patient interface pressure, and the duckbill valve 6090 opens allowing air flow into the patient interface 3000. This air flow will cause the patient interface pressure to increase until the desired CPAP pressure is reached.

The duckbill valve 6090 may open whenever the RPT device pressure (upstream of the valve) exceeds the patient interface pressure (downstream of the valve). In some instances, the valve may be arranged so that it only opens when the upstream pressure exceeds the downstream one by more than a predetermined amount, even though in some cases, the predetermined amount may be very close to zero, so the duckbill valve 6090 again opens almost immediately when the RPT device pressure is greater than the patient interface pressure. If the duckbill valve is designed to open at a predetermined pressure differential, the predetermined pressure differential at which the valve opens can be associated with resistance forces that have to be overcome to part the two lips 6150. Such resistance forces can be related to the mechanical design of the valve, its dimensions and the material of which it is made. Altering these may cause the valve to open at a specific targeted (predetermined) pressure differential. If these resistance forces are negligible, it may be stated that the valve opens as soon as the upstream pressure starts becomes higher than the downstream one.

#### 4.5.1.4 Valve assembly - expiration

Referring next to Figs. 18 and 19, in certain forms of the technology, on expiration the patient interface pressure increases due to the decrease of volume created by the user's diaphragm relaxing (moving up). The pressure sensor 6110 may record this change and signal the RPT device 4000 to reduce or cease air flow. As a result, the patient interface pressure can become greater than the pressure on the RPT device side of the valve assembly 6000 and cause the diaphragm membrane 6100 to move away from the patient interface port rib 6130, introducing an airflow path to atmosphere (i.e. through the vent 6050), as shown in particular in Fig. 19. Simultaneously the duckbill valve 6090 will close, ensuring no reverse airflow travels back into the RPT device. The pressure inside the patient interface 3000 will decrease as the user's expiration progresses and airflow is released to atmosphere. The pressure sensor 6110 will monitor this pressure drop and, in some examples, the RPT device airflow will remain off until the patient interface pressure falls below a predetermined pressure or the set CPAP pressure.

In some examples, the RPT 4000 provides some flow during the expiration phase, even when the patient interface pressure is above a predetermined pressure or the set CPAP pressure. This flow, which may be at a relatively low flow rate, may assist in entraining stagnant $CO_2$. and may therefore improve $CO_2$ washout from the patient interface 3000.

In examples, the pressure differential required to cause the valve membrane to move away from patient interface port rib 6130 (e.g. to initiate venting) is close to zero, such that venting begins almost immediately when the patient interface pressure is greater than the RPT device pressure.

The vent openings 6052 themselves are typically sized so that they have little or no influence on the flow rate through the vent 6050, since the rate of vent flow is controlled by the clearance gap between the diaphragm membrane 6100 and the rib 6130 (the total area of the vent openings 6052 being sufficiently larger than the area for flow between the diaphragm membrane 6100 and the rib 6130 as to have little or no effect on the flow rate). The structure around the vent openings 6052 is provided to provide protection for the diaphragm 6060, protecting it from foreign bodies.

#### 4.5.1.4.1 Duckbill valve - expiration

In certain forms of the technology, when the patient interface pressure is greater than the RPT device pressure, the duckbill valve 6090 will close. Therefore, in examples, no user airflow on expiration will reach the RPT device.

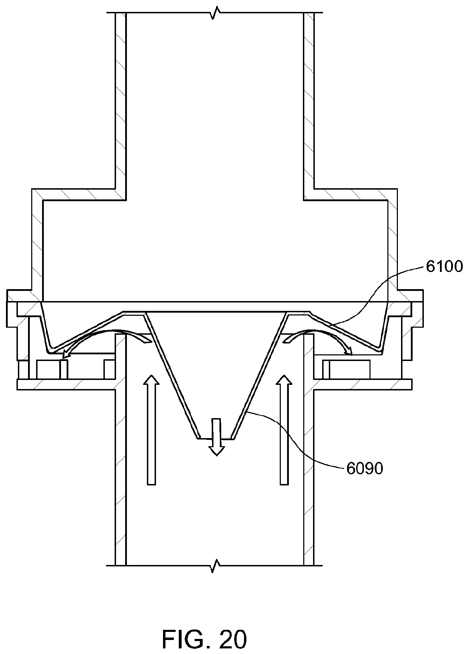

In some cases, as in the example shown in Fig. 20, the valve (i.e. duckbill valve 6090) can be configured to avoid sealing completely when closed, in order to allow some RPT flow to reach the patient interface 3000, even during the expiratory phase. This flow, which may be at a relatively low flow rate, may assist in entraining stagnant $CO_2$, and may therefore improve $CO_2$ washout from the patient interface 3000

#### 4.5.1.5 Diaphragm membrane

Referring next to Figs. 10 and 17, in one form the diaphragm 6060 has a thin membrane portion 6100 connected to the duckbill valve 6090. At or adjacent the duckbill valve 6090 base there is a section, e.g. a small flat section (also referred to as a portion) 6102 of the membrane 6100, that interfaces with the patient interface port rib 6130. In one form the membrane portion 6100 can move up or down in response to the pressure differential acting on both sides. The membrane movement, towards or away from rib 6130, does not have a significant effect on the shape or performance of the outer diameter or duckbill valve 6090.

In examples, the diaphragm 6060 can have a first wall 6140 which extends from the retention flange 6080 to the outer circumference of the membrane portion 6100. In examples having a circular diaphragm, the first wall 6140 may be substantially cylindrical or conical (sloped). In examples where the diaphragm is substantially elliptical, the first wall may be an elliptic cylinder.

In examples, the first wall 6140 extends in a downstream direction from the retention flange 6080, such that the edge of the membrane portion 6100, where the membrane portion 6100 extends from the first wall 6140, is offset in the downstream direction from the retention flange 6080 or the entrance to the patient interface port, e.g. rib 6130. This does not have to be the case and the membrane portion 6100 may be coplanar with either one of retention flange 6080 or rib 6130, or extend upstream from any one of them.

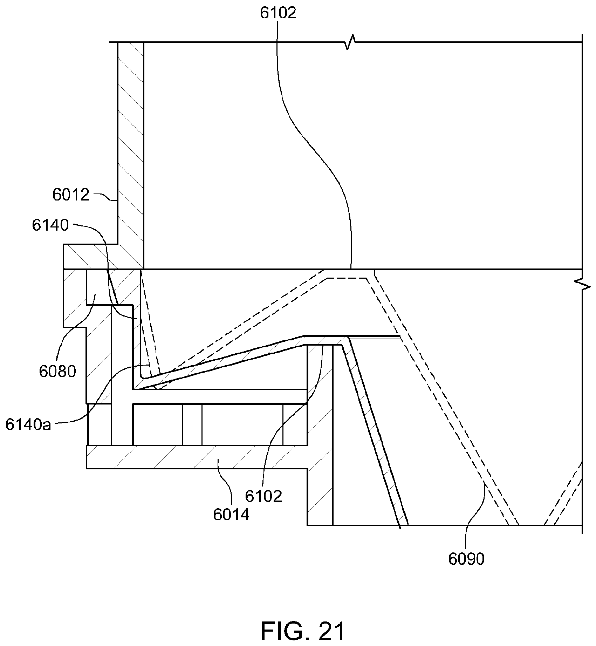

Shaping the diaphragm 6060 in the way shown in the figures, increases the ability of the diaphragm 6060 to move in response to pressure variations. As best seen in Figs. 18-21, when the downstream pressure is greater than the upstream pressure, such that the diaphragm 6060 moves away from rib 6130, the first wall 6140 pivots around the retention flange 6080, to the position indicated by 6140a (Fig. 21). This movement allows the diaphragm 6060 to move away from the patient interface port 6040 without the need for the diaphragm material to stretch, as would be the case if the diaphragm 6060 was substantially planar. In addition, the base of the duckbill valve 6090 may close or at least substantially reduce in width during patient exhalation, thereby increasing the gap between portion 6102 and the annular rib 6130 of the patient interface port, allowing increased outflow of air to vent 6050 and consequently reducing the effort needed to exhale.

Another advantage of the illustrated structure can be related to the capability of the membrane to fulfil its two functions substantially independently. In particular, the decoupling of these two functions may be facilitated by the ability of portions of the membrane to pivot around the retention flange 6080 and or portion 6102. Such pivoting may allow the operation of the central, duckbill-shaped, portion of the membrane essentially as a one-way valve for the airflow generated by the RPT device, to be substantially decoupled from the valve-like function of the outer, more peripheral portions (6102, 6140) of the membrane.

In examples, the first wall 6140 may be substantially the same thickness as the remainder of the diaphragm 6060 (excluding the retention flange 6080). In examples, the diaphragm is configured to be to be as thin and light as possible. Alternatively, portions of the membrane (such as 6140 and/or 6102) may be made thinner than the remaining portions of the membrane, e.g. to improve potential decoupling between respective portions of the membrane.

### 4.5.1.6 Valve assembly - breathing cycle

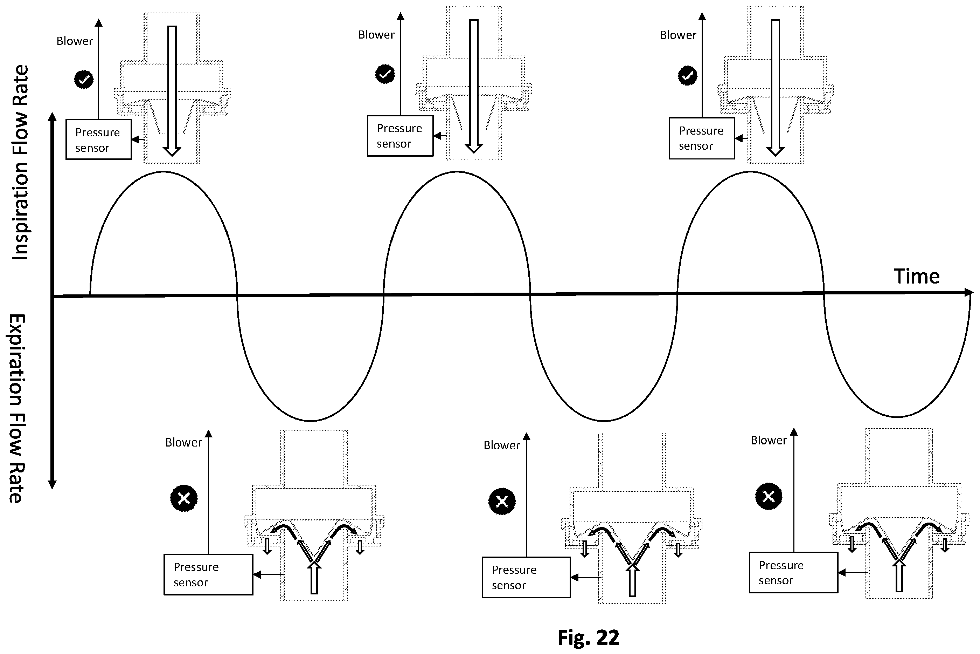

In certain forms of the technology, driven by the pressure differential as described in the above paragraphs, the diaphragm will dynamically move between the inspiration and expiration positions during the user's breathing cycles (see simplified breathing curve illustration in Fig. 22). Because its function is to facilitate an airflow path to ambient atmosphere, the above described valve assembly may be called an expiratory activated valve (EAV). However, as described earlier, another important part of the functionality of the assembly is as a one-way valve that allows airflow generated by the RPT device to reach the mask only (or mostly) during inspiration, whilst limiting or stopping it completely during expiration. Thus the functionality of the assembly is not limited to the expiration stage as it is constantly reacting to all stages of the breathing cycle, with the simplified, exemplary illustrations within this document describing only/mainly the extremes.

### 4.5.1.7 Valve assembly - vent flow

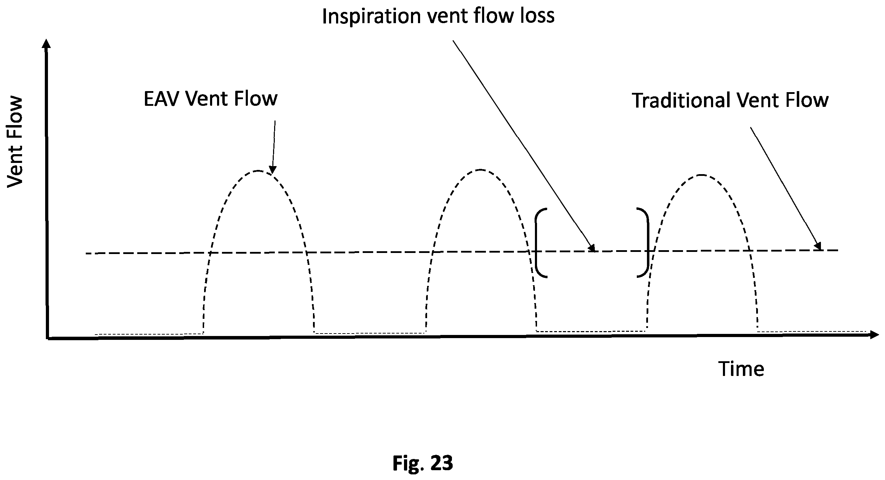

As highlighted in the previous sections of this document, in certain forms of the technology the valve assembly 6000 only vents to atmosphere on expiration. Therefore, the vent flow curve may not follow the constant flow characteristic of a traditional system, but may follow more an 'on-off' curve shape. Fig. 23 shows an example of how the valve assembly 6000 vent flow may look when compared to a traditional system. In such examples, most or all the vent flow that may occur in a valve assembly 6000 system is powered by the user's effort. Since the flow path to the vent is opened only for a portion of the time, the overall loss of pressure/flow, as well as of humidity associated with the lost flow, is reduced. This can reduce the overall demand on the blower and on the humidification system and improve electrical and blower efficiencies when compared to these of a traditional CPAP. Such an improvement in efficiency may allow a smaller blower and/or heater plate for the humidifier, to be used. This would be especially beneficial for portable devices, especially for such that operate on batteries.

Alternatively, the valve member 6020 can be configured so that some limited vent flow to ambient atmosphere is maintained during inspiration, to maintain air flow circulation and a safe $CO_2$ level.

There are certain requirements that the above described valve assembly needs to meet in order for it to be successfully implemented in a PAP system. In accordance with some forms of the present technology, the valve assembly 6000 closes the access to the air circuit 4170 during exhalation. In the absence of access to this internal volume of the air-circuit, most or all of the expired airflow in the present arrangement has to exit to atmosphere through the valve assembly 6000, via the vent 6050. To reduce the risk of pressure increases (swings), such as during expiration, the vent 6050 and the vent openings 6052 of the valve assembly 6000 may need to be configured to allow a high flow rate to reduce the impedance of the flow venting to atmosphere.

In examples of the present technology, such as in the example illustrated in Fig. 19, during the expiration phase the flow path to the vent 6050 is open. This is facilitated by the exhaling pressure creating a gap between the membrane 6100 and the patient interface port rib 6130. The arrangement is at least partially self-regulating, as the gap may increase with the increased patient interface pressure, thereby progressively reducing impedance to flow as the patient interface pressure is increased. The size of the opening created by the membrane being moved away from rib 6130 is limited by the resistive force created by the resilient nature of the membrane, which will resist the membrane deflection away from its equilibrium state. Because of this regulating mechanism, the patient is faced with a reduced impedance during exhalation with generally, the higher the breathing effort, the larger the reduction in breathing impedance. Thus the opening of the vent flow path during exhalation makes breathing out easier for the patient, and reduces the pressure swing (the variation in patient interface pressure) during exhalation. By contrast, patient interfaces of the prior art which have a fixed venting area may experience increased patient interface pressure during peak expiratory flows. Thus, this valve assembly can bring some substantial benefits when used with a PAP system. This is even more important when one considers the fact that the user of such a system is often asleep and sudden pressure swings may wake the user up. A self-regulating system that reduced these pressure swings by dynamically adjusting the size of the airflow path to ambient atmosphere, may help avoid such episodes, thus improving the user comfort and potentially improving the compliance with the prescribed treatment.

In examples of the present technology, the patient interface 3000 (and/or the valve) may be configured to allow a small vent flow (e.g. 0.1 L/s) during the inspiration phase, e.g. when the membrane is closed against the patient interface port rib 6130. Such a vent flow may be much smaller (e.g. around 10%) of the vent flow of a prior art patient interface. Such a small vent flow may assist with CO2 washout, whilst still reducing the power requirements to the blower and to the humidification system.

The structure and function of the valve is best shown in Fig. 24 that also shows simulated flow through duckbill valve 6090 according to one form of the technology. As can be seen from Fig. 24, the ends 6160 of the two lips 6150 are connected together, such that when the duckbill valve 6090 is open the lips 6150 are not parallel, but rather form a vesica piscis-type or lens-type of shape opening 6170.

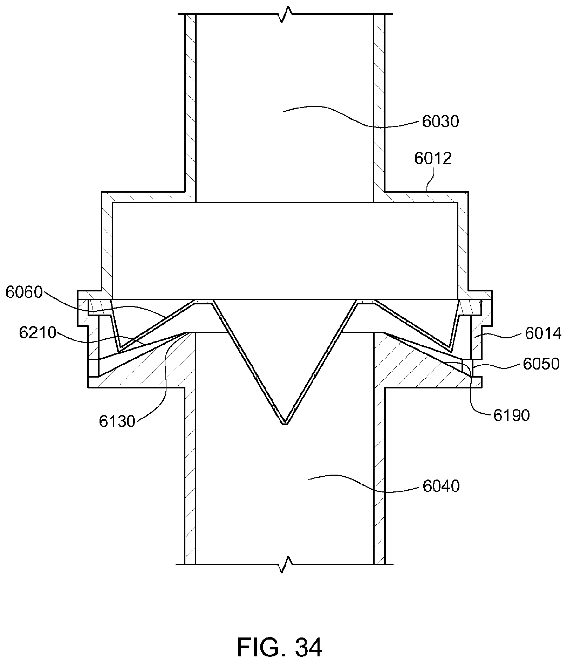

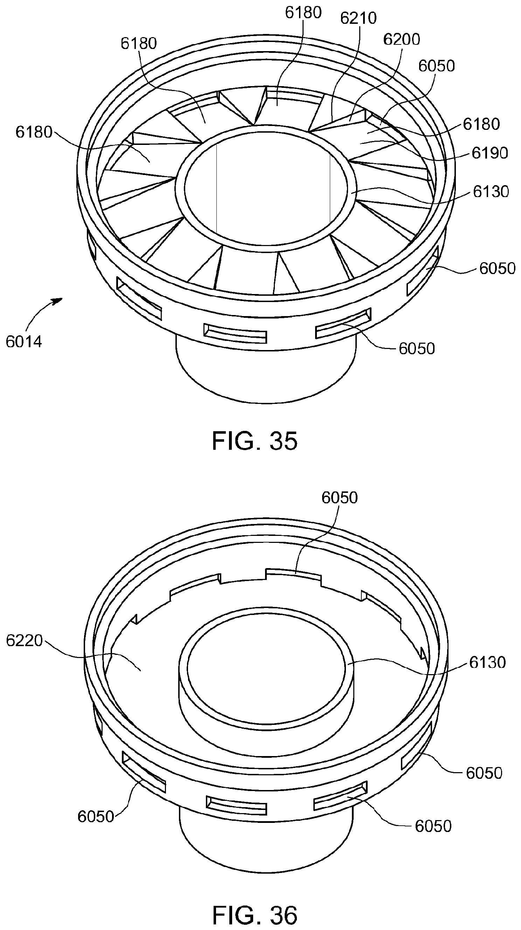

Referring next to Figs. 34 and 35, in some examples the downstream housing portion 6014 may be provided with one or more flow guide portions 6180 which guide the exhalation flow from the inlet of the patient interface port 6040 (e.g. rib 6130) to one or more respective ambient ports/vents 6050 during expiration.

In examples, there are a plurality of the flow guide portions, each of which comprises a ramp portion 6190. In examples, each ramp portion 6190 slopes slightly downward from the level of the edge 6130, where the airflow leaves the mask port 6040 (during exhalation), towards the lower edge of the opening of each respective port 6050.

The flow guide portions 6180 may comprise side wall portions 6200 on either side of each ramp portion 6190 such that a channel is formed. In some examples the upper edge 6210 of each side wall portion 6200 may be level, or may slope from the level of the top surface of a respective ambient port 6050 to approximately the level of the rib 6130. The slope may be upward or downward. The arrangement is such that the side wall portions 6200 do not interfere with the closing of the diaphragm 6060.

The flow guide portions 6180 may assist in reducing the turbulence in the flow to the ambient port 6050 (e.g. during expiration) by avoiding, or at least reducing the magnitude of, any sudden expansion of the expired gas. The gas may instead propagate proximate to the flow guide surfaces.

By contrast, the arrangement of the downstream housing portion 6014 of the examples shown in Figs. 17 to 21 and Fig. 36 is such that during expiration, the airflow which has passed through the relatively narrow gap between the rib 6130 and membrane section 6102 may need to flow around sharp edges, make sharp turns, and suddenly expand into a relatively large chamber 6220 (see Fig. 36), and may therefore become turbulent.

Turbulent flow between the rib 6130 and the ambient ports 6050 may cause vibrations in the membrane 6100, which may cause variations in the clearance between the rib 6130 and membrane section 6102 and may in turn cause variations in the vent flow. The vibrations may also be associated with an audible effect, which may be uncomfortable to the user. By contrast, the smoother vent flow created by the arrangement of Figs. 34 and 35 examples having flow guides 6180 may be smoother, steadier and quieter. Such a flow is also closer to laminar flow, which may be more accurately estimated based on the models and methods described herein.

### 4.5.2 Reducing Noise

Due to the constant dynamic readjustment of the configuration of the above described valve during the respiratory cycle of a user, the membrane may generate a flap-like (or thump-like) noise often present in membrane valves. This noise may be uncomfortable for the user and interfere with the user's sleep during the prescribe therapy.

In examples, such a noise can be reduced if the valve member 6020 is made from relatively soft material, such silicone or silicone rubber. Alternative soft materials may also be used. Thus, manufacturing the valve member 6020 from silicone or silicone rubber may, among other advantages, reduce the noise created when the duckbill portion 6090 of the valve closes. Manufacturing the valve member 6020 from other materials (particularly harder materials) may result in a flapping (thumping) noise being generated each time the two opposing portions of the duckbill valve 6090 come into contact when the valve closes, as well as when the portion 6102 contacts the rib 6130.

The use of an elongate shaped valve member 6020, e.g. elliptical or stadium shaped valves (for example as shown in Figs. 11 and 12) and correspondingly shaped duckbill valves 6090 may also result in reduced noise generation, compared to circular or disc shaped valves. Because of the elongated shape, even a small movement of the two opposite lips 6150 of the duckbill valve 6090 in a direction transverse to the length of the "duckbill", creates a large overall opening. This, in turn, reduces the distance the parts 6150 need to travel to create a given flow area, when moving between an open position and a closed position. The maximum speed achieved by the parts 6150 during their movement is also reduced. The reduced distance and speed may reduce the overall noise associated with the movement.

As best seen in Figs 11 and 12, in examples, the base of the duckbill valve 6090 may have a length dimension L, which is substantially parallel to a major axis of the diaphragm 6060, and a width dimension W, which is substantially parallel to the minor axis of the diaphragm 6090 (the minor and major axes being transverse to each other). To achieve the noise-reduction benefit of an increased length of the valve, the length L may be greater than the width W. In examples the ratio of L:W may be at least 1.5:1, for example around 2:1. In examples the ratio of the length of the major axis of the diaphragm 6090 to the length of the minor axis of the diaphragm 6090 is a least 4:3.

Measures taken to reduce resonance in the diaphragm (for example as set out below) may also reduce noise.

### 4.5.3 Reducing Resonance

In examples, and as discussed in previous paragraphs, periodic variations in the pressure differential across the valve membrane 6100 (including, for example, variations caused by adjustments to the blower flow rate) may cause the membrane 6100 to vibrate at a dominant resonant frequency associated with the structure and the configuration of the membrane. This may create an undesirable noise and/or may affect the operation of the diaphragm.

To reduce the chances of resonance occurring during normal operation of the device, the valve member 6020 may be formed with one or more portions or regions having a different thickness to one or more other portions or regions. Each portion may therefore have a different resonant frequency, such that there is no one single dominant resonant frequency for the entire membrane. In examples, an arrangement is targeted where a resonant response may only occur under conditions which are outside the expected operating conditions of the system (e.g. in a system which is intended for use up to a maximum pressure of 20 cmH2O, the system may be configured such that a resonant response would be expected if the system was used at a pressure of 24 cmH2O).

In some cases, the change in the resonant response may be changed by way of varying the shapes or structure of the membrane 6100 (e.g. the areas of varying thickness) into an arrangement where the membrane does not have an axis of symmetry, has only a single axis of symmetry, or has no more than two axes of symmetry. Any of the above arrangements may change the membrane's resonant behaviour and reduce the overall noise associated with the resonance of the membrane.

In one example, the membrane 6100 may comprise one or more areas or sections characterised by one or more different material properties or features. One example of that may be a variable (e.g. reduced) thickness which can accelerate and decelerate (and hence open and close) faster than the other area(s) of the membrane. Instead of two dimensional (e.g. relatively broad) areas of reduced thickness being provided as a way of altering the resonance response of the membrane 6100, the membrane 6100 may have one or more physical features (e.g. say one or more substantially one-dimensional line of reduced thickness, which can be also referred to as grooves and/or cuts. Such different areas of different thickness or one or more grooves, may define one or more portions of the membrane that are able to move at least partially independently with respect to other portions. Some of these portions may be arranged to open/flap more easily than the rest or to get involved in the opening/closing of the flaps at an earlier/later stage compared to others. This may also mean that one or more of these portions may resonate at different time or at different frequencies compared to the remaining portions, bringing about a more complex resonant behaviour where the membrane 6100 does not resonate as a single body and at a single frequency. This may reduce the overall noise associated with the resonance movement of the membrane. In an alternative arrangement, instead of grooves, one or more stiffening ribs may be introduced on the surface, or within the body of the membrane. The ribs may also partition the membrane, create a non-symmetrical structure and modify the resonant behaviour of the membrane, potentially reducing the overall noise.

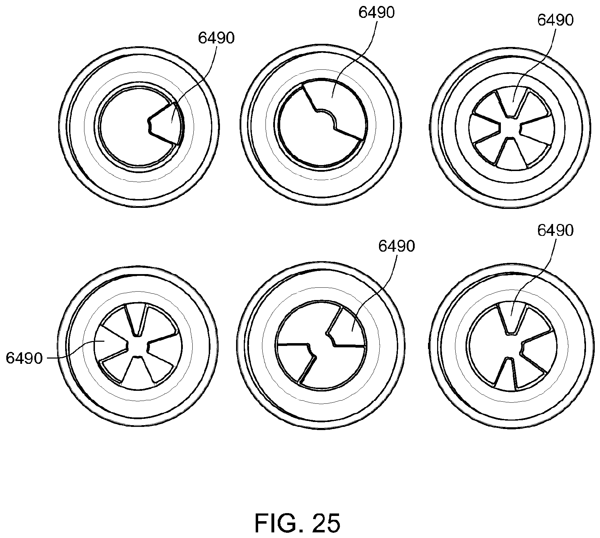

By way of illustration, Fig. 25 shows examples of other forms of valve members, which are not provided with duckbill valves, but which have exemplary portions of different thickness, in this case areas 6490 of thickness that in this case is reduced, but may also be increased, when compared to the main body of the membrane 6100. Although not shown in Fig. 25, a combination of more than 2 different thicknesses - e.g. where one or more regions have a reduced, and one or more regions have an increased thickness, compared to the main body of the membrane, are also possible. Similarly, areas of different thickness may be introduced in the context of a duckbill arrangement.

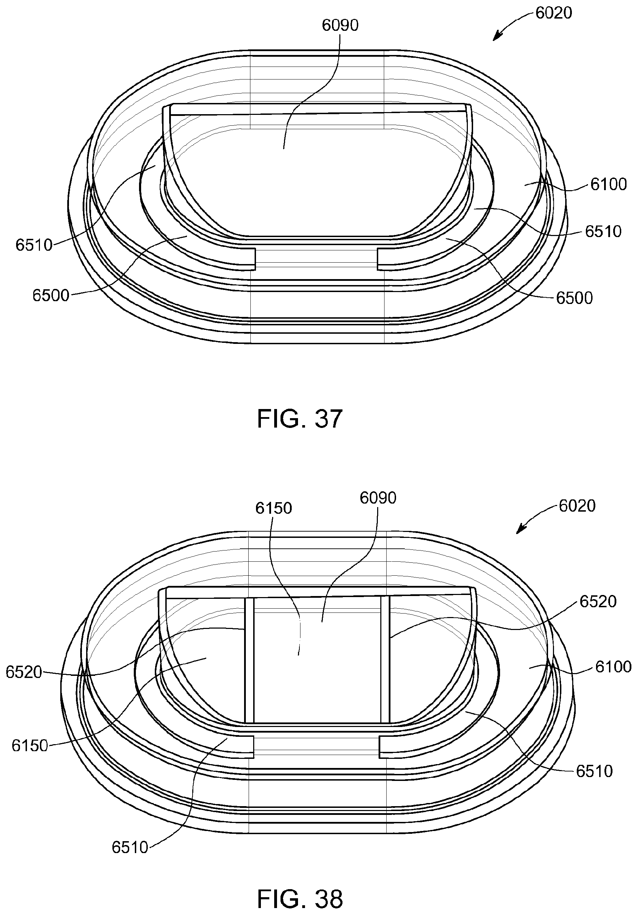

Referring next to Fig. 37, in one example the resonant response of the outer peripheral portion (e.g. the membrane 6100) of the diaphragm 6060 may be varied by adding one or more thickened portions 6500. In the example shown, the thickened portion 6500 is in the form of a substantially "C" shaped thickened portion 6510 provided adjacent an outer perimeter of the duckbill valve 6090.

Referring next to Fig. 38, in another example areas of increased thickness may be provided to the two opposing lips 6150 of the duckbill valve 6090, if this is where the targeted response and resulting vibrations occur. In the example shown, the areas of increased thickness are provided as narrow lines of material 6520 which extend from a base of each lip, say to the edge of the respective lip 6150. As with the example shown in Fig. 37, the thickened portions 6510, 6520 may be integrally formed with the rest of the valve member 6020, or they may comprise separately formed components which are connected to the valve member 6020. As well as affecting the mass of the component to which they are attached, areas of increased or decreased thickness may also alter the stiffness of the component and/or introduce an asymmetrical response to an otherwise symmetrical part.

Although Fig. 38 shows an example with thickened portions 6510 on the membrane 6100 in combination with thickened portions 6520 on the lips 6150 of the duckbill valve 6090, in other examples the duckbill valve 6090 may have thickened portions and the membrane 6100 may not, depending on where the target resonance and vibrations are.

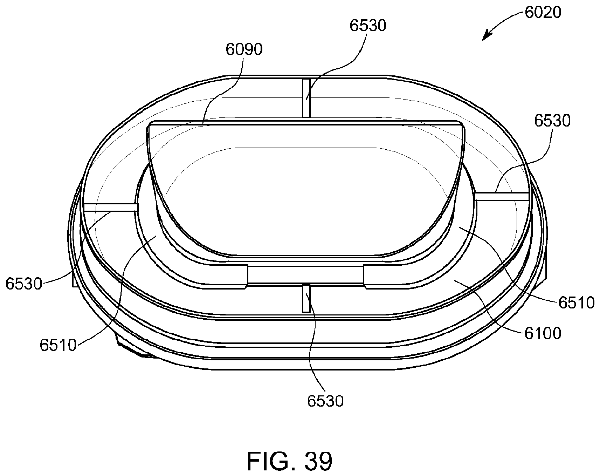

Fig. 39 shows another example of the technology having the C-shaped thickened portions 6510 shown in Fig. 37 in combination with linear thickened portions 6530 which extend radially across the membrane 6100. In the example shown there are four radially extending thickened portions 6500, but in other examples more or fewer may be provided. In other examples the C-shaped portions 6510 may be omitted.

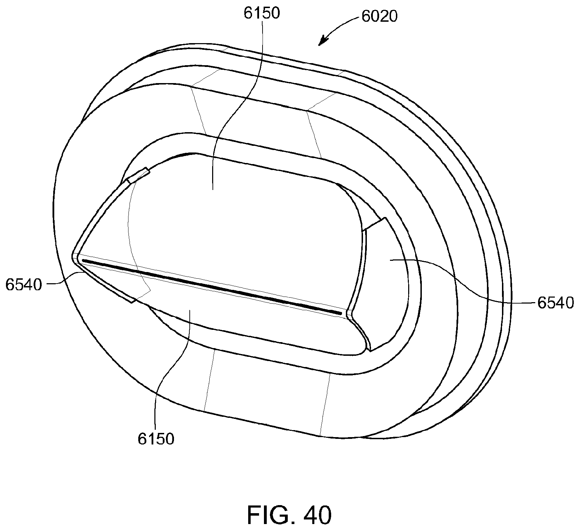

Fig. 40 shows another example having thickened portions 6540 provided to the sides of the duckbill valve 6020. In this example the thickened portions 6540 are not provided to the lips 6150 themselves. The thickened portions 6540 may have a thickness which is twice that of the thickness of the lips 6150.

Each of the thickened portions described above may be integrally formed with the rest of the valve member 6020, or may comprise a separately formed component which is connected (removably or otherwise) to the valve member 6020. The separately formed component may be formed from the same material as the membrane 6100 or duck bill valve 6090, or a different material. In the case where a different material is used, the density and/or stiffness of the material forming the separate element may be different from that used to form the adjacent or underlying portions of the valve member 6020.

In examples, the difference in thickness between thickened or thinner portions 6490, 6500-6540 relative to the adjacent portions of the valve member 6020 may vary from about a 1.5:1 ratio, to much larger ratios, e.g. 4:1 or even 8:1 or 10:1 ratios). For example, if the majority of the membrane 6100 has a thickness of about 0.5 mm, the thickened portions 6500 may be about 2 mm thick. Similarly, if the majority of the membrane 6100 has a thickness of about 4 mm, thinner portions 6490 of about 3 mm thickness may be provided.

As mentioned above, in other examples, additional elements may be overlaid over the valve member 6020 to change its resonant characteristics. Such elements may not be connected to the valve member 6020 over their entire length. In one example, such elements may comprise a frame which is only fixed to a respective portion (the membrane 6100 or a respective lip 6150) of the valve member 6020 at its outer periphery, or which is not fixed to the valve member at all, but is mounted such that at least some movement of the valve member 6020 interferes with the frame.

In other examples, the valve member 6020 may have portions made from materials having different material properties. For example, one portion of the valve member 6020 may have a different density and/or rigidity as compared to another portion of the valve member 6020. In some examples, different portions may be made from different materials. In examples the diaphragm 6060 may have a plurality of portions with differing material properties.

Creating variations in the thickness of various portions of the membrane 6100 is a relatively low cost process that can help improve the resonant response of the membrane. Using materials with different property for various portions of the membrane or connecting/attaching additional components to such various portions may result in increased costs and manufacturing difficulty. However, these methods may still be applicable in some circumstances. For example, adding a C-shaped insert 6510 may be a preferred option to mitigate the resonant response of an existing membrane, such as membrane 6100 (Fig. 37) as it can be relatively easily retrofitted to a bellow channel of the membrane.

While various resonance reducing methods have been described above with reference to examples of stadium shaped valve members 6020, the same methods may also be used with other shapes of valve members, e.g. circular valve members. Similarly, whilst various lines and areas have been described as examples of areas with different thickness and/or rigidity, other shapes and forms may also be used.

4.6 VENT FLOW ESTIMATION

As set out above, in examples, the RPT device 4000 may comprise an apnea detection algorithm.

In order to detect an apnea, the RPT device 4000 must be capable of measuring or estimating the patient's respiratory flow rate during inspiration and expiration.

Direct measurement of patient respiratory flow may be difficult or impossible. Therefore (as with most prior art systems), the system may rely on measurement of the flow rate ($Q_B$) generated by the blower to derive the patient flow.

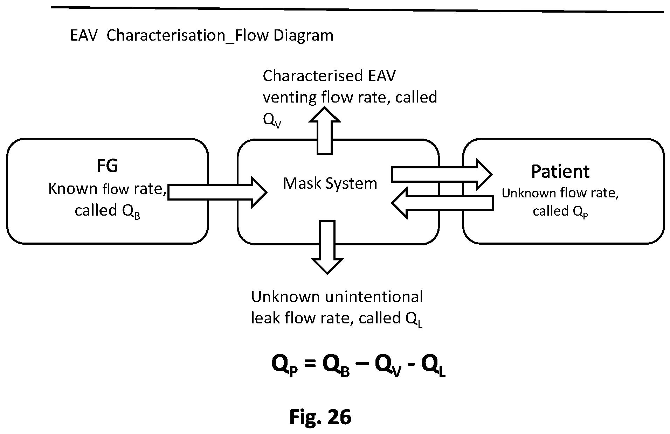

To calculate patient flow rate ($Q_p$), the system must account for losses due to venting (i.e. intentional leak Qv) and unintentional leak ($Q_l$) when deriving the patient flow, as shown in Fig. 26, in order to estimate the flow rate ($Q_p$) provided to the patient.

In the systems of the prior art which have a constant area for venting, the vent flow Qv can be estimated relatively simply based on the pressure within the patient interface 3000, which can in turn be estimated accurately from the pressure at the outlet of the blower 4142. However, such prior art algorithms may not accurately estimate the vent flow Qv from the valve assembly 6000 of the present invention due the ability of the valve 6000 to dynamically alter the vent flow opening in response to the pressure differential across the diaphragm 6060. This pressure differential is caused, in part, by the patient's breathing.

The applicant has found that in one or more examples of the present technology, the vent flow Qv can be accurately estimated across a useful range of pressures based on one or more ratios between the pressures on both sides of the membrane (namely the blower pressure (Bp) and patient interface pressure (Pp)).

### 4.6.1.1 Method of characterising the vent flow

In order to estimate the vent flow Qv, the flow to the vent must be mathematically modelled.

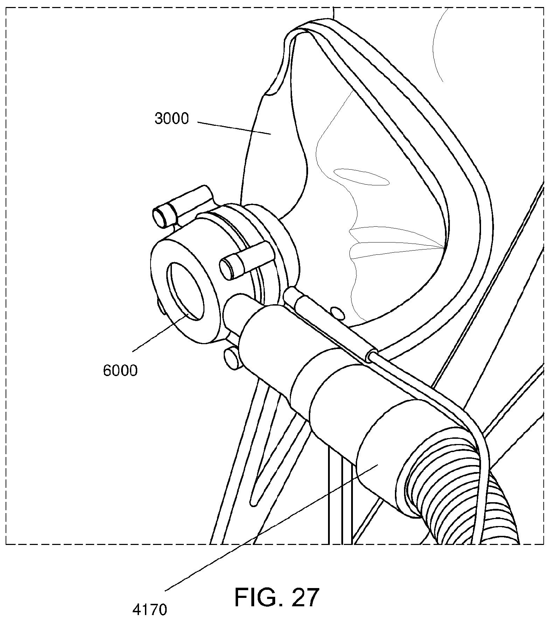

One example system including an EAV, such as the one discussed in relation to Figs. 8-24, with a downstream housing having a structure as shown in Figs. 34 and 35, is shown in Fig. 27.

An attempt to conduct a static characterisation (i.e. at various constant pressure/flow operating points) of the valve assembly 6000 in Fig. 27 indicated that the EAV presents some hysteresis. Because of that, it was decided to pursue a dynamic data collection and analysis using various simulated breathing patterns, at various therapy pressures. Various parameters associated with the valve assembly 6000 system were measured/estimated during one or more simulated dynamic breathing cycles. The measured/estimated parameters included at least blower pressure (Bp), patient interface pressure (Pp) and vent flow rate (Qv). The goal of running a characterization during a breathing cycle is to capture the characteristic of the valve when subjected to a range of conditions. Because of that, the simulated breathing cycle parameters were selected to provide a large range of respiratory rates, peak expiratory flows and tidal volumes. The chosen variety of simulated breathing characteristics facilitated the analysis of the valve behaviour for at least one of a) one or more flow rates; b) one or more flow accelerations and/or decelerations; c) one or more peak expiratory flows; d) one or more tidal volumes etc.

The test was repeated for a plurality of different mask pressures, e.g. 4 cmH2O, 6 cmH2O, 8 cmH2O etc.

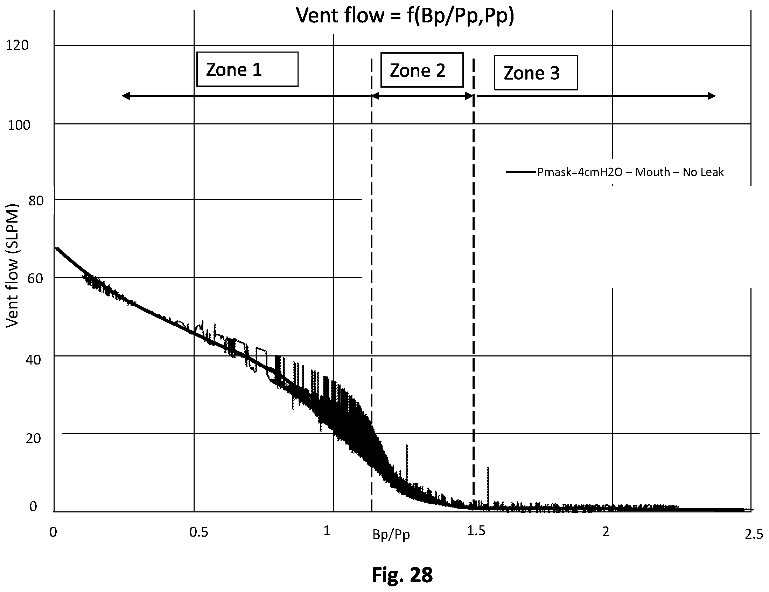

The applicant has discovered that in all of the above cases, vent flow Qv can reliably be characterised as a function of pressure ratio between the pressures on both sides of the membrane (e.g. Bp/Pp). Vent flow vs pressure ratio (Bp/Pp) was plotted for each therapy pressure. For a better characterisation, each plot was divided into a plurality of regions or zones (e.g. three zones), according to the trends exhibited by the data in the overall graph. In one example (shown in Fig. 28):

- Zone 1 is where Bp/Pp is less than 1.2;

- Zone 2 is where Bp/Pp is between 1.2 and 1.5; and

- Zone 3 is where Bp/Pp is greater than 1.5.

Next, an equation for the best fit curve in each zone was derived using standard numerical methods. The applicant has found that equations of different orders may be preferred for one or more of the different zones. For each zone, increasingly higher order equations were trialled until the improvement in fit to the data stopped improving.

In one form of the technology:

- Best fit to the data in Zone 1 was found to be a 3rd order polynomial equation with respect to the Bp/Pp ratio;

- Best fit to the data in Zone 2 was found to be a 2nd order polynomial equation; and

- Best fit to the data in Zone 3 was found to be a linear equation.

Thus, in one form of the technology, characterised by the specific configuration of the valve described here (see Fig.27), air circuit and patient interface used, the mask vent flow could be estimated using the following equations:

Zone 1:

$$\text{Vent flow} = a * (Bp/Pp)^3 + b * (Bp/Pp)^2 + c * (Bp/Pp) + d$$

Zone 2:

$$\text{Vent flow} = a * (Bp/Pp)^2 + b * (Bp/Pp) + c$$

Zone 3:

$$\text{Vent flow} = a * (Bp/Pp)$$

The applicant has also found that the boundaries defining each zone may differ depending on the therapy pressure.

In order to determine suitable polynomial coefficients and boundaries between zones for therapy pressures other than those for which tests had been performed, linear interpolation was used.

Table 1 below shows the fitted equation coefficients and zone boundaries for two measured therapy pressures (4 cmH2O and 6 cmH2O), as well as interpolated equation coefficients and zone boundaries for a variety of therapy pressures between these two pressures (4 cmH2O and 6 cmH2O), for one form of the technology.

## Table 1 - polynomial coefficients and zone boundaries

| Pmask | Zone 1- 3rd order polynomial coefficients | | | | Zone 2 - 2nd order polynomial coefficients | | | Zone 3 - constant | 1 to 2 transition | 2 to 3 transition |
| | a | b | c | d | a | b | c | a | | |
|---|---|---|---|---|---|---|---|---|---|---|
| 4 | -39.8615 | 62.619 | -65.7738 | 68 | 117.49 | -343.22 | 252.18 | 0.8 | 1.2 | 1.5 |
| 4.1 | -39.6035 | 60.6517 | -65.1794 | 68.6 | 113.5535 | -331.559 | 243.5405 | 0.8 | 1.195 | 1.495 |
| 4.2 | -39.3456 | 58.6844 | -64.585 | 69.2 | 109.617 | -319.897 | 234.901 | 0.8 | 1.19 | 1.49 |
| 4.3 | -39.0876 | 56.7171 | -63.9906 | 69.8 | 105.6805 | -308.236 | 226.2615 | 0.8 | 1.185 | 1.485 |
| 4.4 | -38.8296 | 54.7498 | -63.3962 | 70.4 | 101.744 | -296.574 | 217.622 | 0.8 | 1.18 | 1.48 |
| 4.5 | -38.5716 | 52.7825 | -62.8019 | 71 | 97.8075 | -284.913 | 208.9825 | 0.8 | 1.175 | 1.475 |
| 4.6 | -38.3137 | 50.8152 | -62.2075 | 71.6 | 93.871 | -273.251 | 200.343 | 0.8 | 1.17 | 1.47 |
| 4.7 | -38.0557 | 48.8479 | -61.6131 | 72.2 | 89.9345 | -261.59 | 191.7035 | 0.8 | 1.165 | 1.465 |
| 4.8 | -37.7977 | 46.8806 | -61.0187 | 72.8 | 85.998 | -249.928 | 183.064 | 0.8 | 1.16 | 1.46 |
| 4.9 | -37.5397 | 44.9133 | -60.4243 | 73.4 | 82.0615 | -238.267 | 174.4245 | 0.8 | 1.155 | 1.455 |
| 5 | -37.2818 | 42.946 | -59.8299 | 74 | 78.125 | -226.605 | 165.785 | 0.8 | 1.15 | 1.45 |
| 5.1 | -37.0238 | 40.9787 | -59.2355 | 74.6 | 74.1885 | -214.944 | 157.1455 | 0.8 | 1.145 | 1.445 |
| 5.2 | -36.7658 | 39.0114 | -58.6411 | 75.2 | 70.252 | -203.282 | 148.506 | 0.8 | 1.14 | 1.44 |
| 5.3 | -36.5078 | 37.0441 | -58.0467 | 75.8 | 66.3155 | -191.621 | 139.8665 | 0.8 | 1.135 | 1.435 |
| 5.4 | -36.2499 | 35.0768 | -57.4523 | 76.4 | 62.379 | -179.959 | 131.227 | 0.8 | 1.13 | 1.43 |
| 5.5 | -35.9919 | 33.1095 | -56.858 | 77 | 58.4425 | -168.298 | 122.5875 | 0.8 | 1.125 | 1.425 |
| 5.6 | -35.7339 | 31.1422 | -56.2636 | 77.6 | 54.506 | -156.636 | 113.948 | 0.8 | 1.12 | 1.42 |
| 5.7 | -35.4759 | 29.1749 | -55.6692 | 78.2 | 50.5695 | -144.975 | 105.3085 | 0.8 | 1.115 | 1.415 |
| 5.8 | -35.218 | 27.2076 | -55.0748 | 78.8 | 46.633 | -133.313 | 96.669 | 0.8 | 1.11 | 1.41 |
| 5.9 | -34.96 | 25.2403 | -54.4804 | 79.4 | 42.6965 | -121.652 | 88.0295 | 0.8 | 1.105 | 1.405 |
| 6 | -34.702 | 23.273 | -53.886 | 80 | 38.76 | -109.99 | 79.39 | 0.8 | 1.1 | 1.4 |

EP 4 387 511 B1

The derived formulas, coefficients and zone boundaries are likely to be dependent on the specific membrane and overall valve system configuration, as well as on the mask space and configuration and the conduit space and configuration. However, once they are derived for the particular combination of mask, conduit and valve system, they are likely to cover a large range of pressures and flows. The coefficients can also be pre-calculated and included in a table similar to the above Table 1. Thus, during the operation of the RPT device, instead of constantly calculating these coefficients, the processor may refer to the tabulated numbers. This can substantially improve the computational efficiency and reduce the demand to the processor.

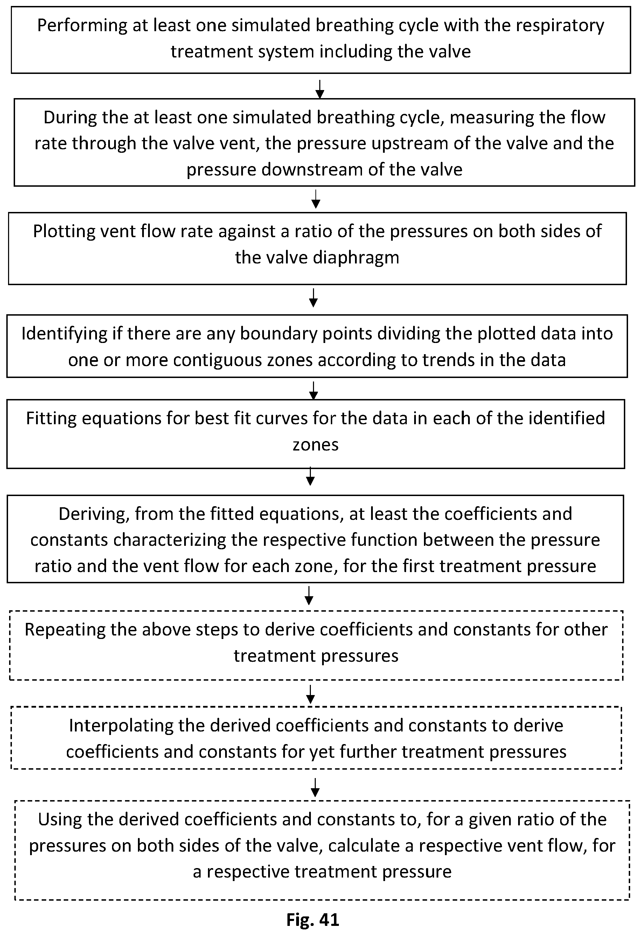

A summary of the method of characterising the vent flow is shown in Fig. 41, and includes the following steps:

a. performing at least one simulated breathing cycle with the respiratory treatment system (step 8000);
b. during the at least one simulated breathing cycle, measuring the flow rate through the valve vent, the pressure upstream of the valve and the pressure downstream of the valve (step 8010);
c. plotting vent flow rate against a ratio of the pressures on both sides of the diaphragm (step 8020);
d. identifying if there are any boundary points dividing the plotted data into one or more contiguous zones according to trends in the data (step 8030);
e. deriving equations for best fit curves for the data in each of the identified zones (step 8040); and
f. deriving, from the fitted equations, at least the coefficients and constants characterising the respective function between the pressure ratio and the vent flow for each zone, at least at the first treatment pressure (step 8050).

Optional steps are also shown, including:

• repeating steps a) to e) for at least one second treatment pressure, to derive, from the respective fitted equations, the coefficients and constants characterising the respective function for each zone at the at least one second treatment pressure (step 8060);
• interpolating the derived coefficients and constants to derive further coefficients and constants for treatment pressures other than the at least first and second treatment pressure (step 8070); and
• using the derived and/or the interpolated coefficients and constants to, for a given ratio of the pressures upstream and downstream of the valve, calculate a respective vent flow, for one or more respective treatment pressures (step 8080).

Using the above method of calculating vent flow Qv, and existing methods of measuring and/or calculating the blower flow $Q_B$ and the unintended leak flow $Q_L$, the patient flow Qp can be calculated using the formula in Fig. 26.

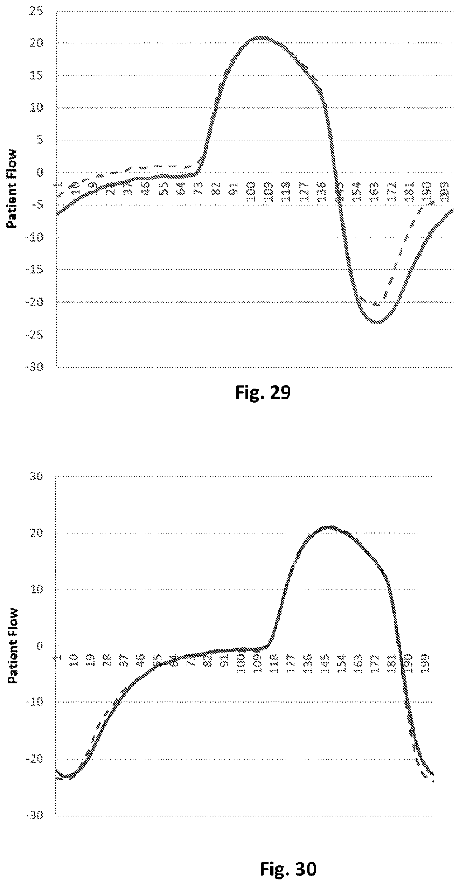

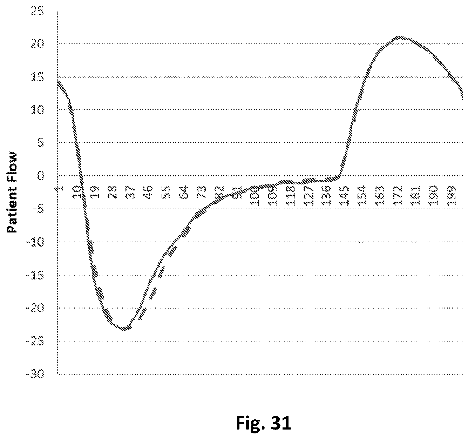

Figs. 29, 30 and 31 show experimentally measured flow rates (solid lines) and estimated flow rates (broken lines) for dynamically varying patient flow rates Qp at three different therapy pressures, 6 cmH2O (Fig. 29), 8 cmH2O (Fig. 30) and 12 cmH2O (Fig. 31). These figures demonstrate that the method set out above allows accurate estimation of patient flows. The reference to dynamically varying flow rates is intended to indicate the fact that, instead of using a fixed pressure and/or flow rate, the graphs reflect a dynamic system that covers a range of pressures and flow rates typically associated with a patient respiratory cycle during the use of the RPT device.

The application of the above described valve assembly 6000 in PAP devices brings specific advantages of improved usability and increased efficiency and performance to the PAP system. The improved efficiency may allow the design of portable and more energy and space efficient systems. The self-regulating nature of at least some functionalities of the valve can also be useful in a system operating on the basis of a closed control loop that maintains the pressure at the user's airways at a target pressure or within a target range of pressures. However, because of the different structure and functionality of the valve assembly 6000, known methods of characterising the system may no longer be suitable, or even applicable. The ability to reliably derive the vent flow of the valve assembly allows for the user flow to be calculated, thus allowing all associated therapy functions such as triggering, cycling, autoset algorithms, flow limitation detection etc. to be performed. This facilitates the practical implementation of a PAP systems using such dual function valve assemblies and allows new and improved systems, including portable systems of reduced size, to be developed.

It should be noticed that some obvious alternatives will also fall within the scope of the above disclosure. For example, whilst the above description refers to plotting the vent flow vs pressure ratio (Bp/Pp) for a number of therapy pressures, the flow may alternatively be plotted against the inverse pressure ratio (Pp/Bp). Such a plot will allow similar subsequent analysis and formula/parameter derivation as the one described above.

4.7 HUMIDIFIER

**4.7.1 Humidifier overview**

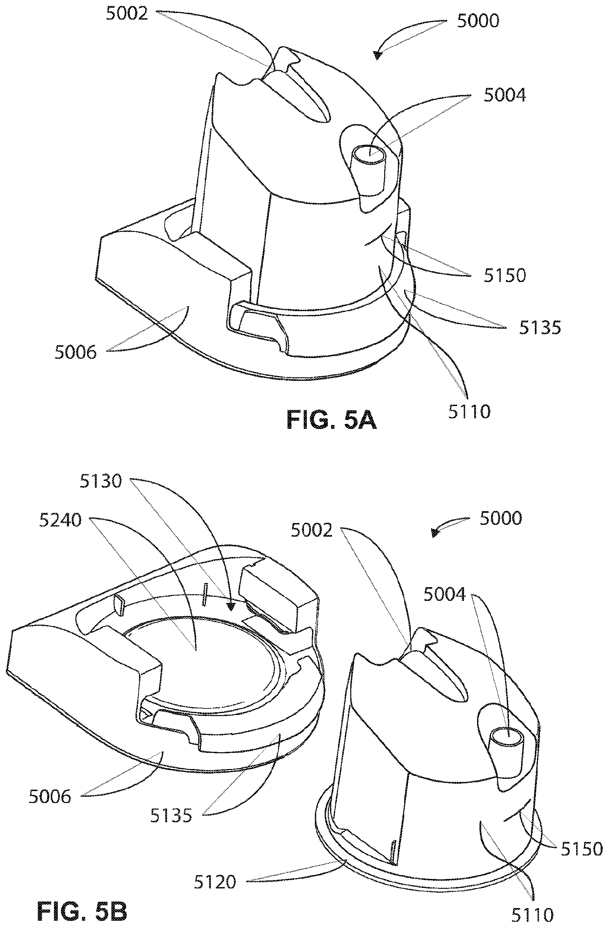

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a dock 5130, a humidifier reservoir 5110, a humidifier inlet 5002 to receive a flow of air, and a humidifier outlet 5004 to deliver a humidified flow of air. In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

In examples the humidifier further comprises a conductive portion 5120, a locking lever 5135 and a water level indicator 5150.

In one form of the present technology, an anti-spill back valve 4160 is located between the humidifier 5000 and the pneumatic block 4020.

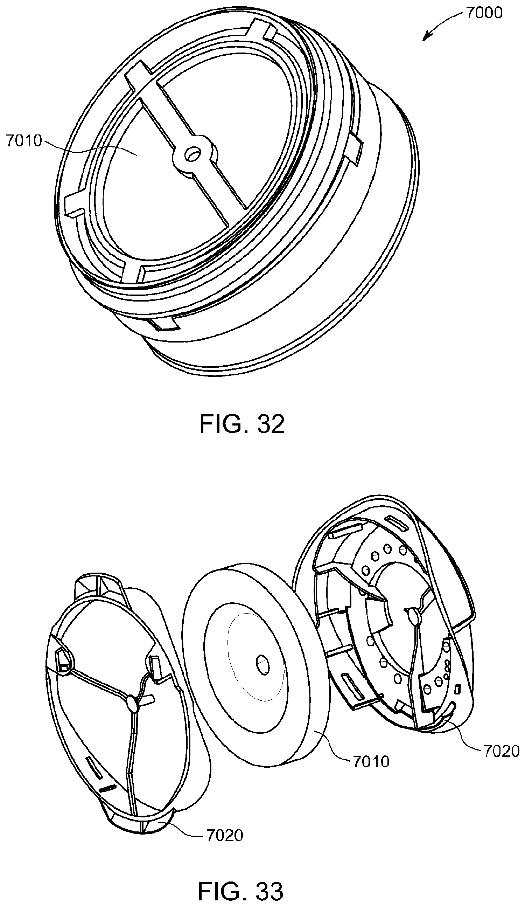

Another form of humidifier in accordance with the present technology is a passive or condensing humidifier, which may also be referred to as a heat and moisture exchanger (HMX) 7000. Exemplary forms of passive humidifiers are illustrated in Figures 32 and 33. The passive humidifier 7000 may comprise a hygroscopic material 7010 held in place between two frame members 7020. In use the passive humidifier 7000 may be located in the air circuit 4170 between the patient interface 3000 and the valve assembly 6000 to collect moisture exhaled by the patient.

In examples of the invention, one consequence of reducing the vent flow during the inspiration phase is a reduction in the loss of moisture/humidity from the system compared to some patient interfaces of the prior art. Because in these prior art systems the vent assembly vents to atmosphere during inspiration, previously exhaled air can leave the system, causing a potentially appreciable amount of airflow (and therefore pressure) and humidity to be lost to atmosphere. In contrast, the above described vent arrangement is closed to ambient during inspiration and most of the moisture/humidity included in the exhaled air and created by the humidifier 5000 goes to the patient's lungs. This may reduce the amount of humidity a humidifier such as that shown in Figs. 5A and 5B must provide, thereby reducing the amount of water and energy consumed by such a humidifier. Reduced vent flow during inspiration may also assist a passive humidifier 7000 such as that shown in Figs. 32 and 33 with providing sufficient humidity to the patient, even in the absence of added moisture from a humidifier such as that shown in Figs. 5A or 5B.

## 4.8 BREATHING WAVEFORMS

Fig. 6 shows a model typical breath waveform of a person while sleeping. The horizontal axis is time, and the vertical axis is respiratory flow rate. While the parameter values may vary, a typical breath may have the following approximate values: tidal volume $Vt$ 0.5L, inhalation time $Ti$ 1.6s, peak inspiratory flow rate $Qpeak$ 0.4 L/s, exhalation time $Te$ 2.4s, peak expiratory flow rate $Qpeak$ -0.5 L/s. The total duration of the breath, $Ttot$, is about 4s. The person typically breathes at a rate of about 15 breaths per minute (BPM), with Ventilation $Vent$ about 7.5 L/min. A typical duty cycle, the ratio of $Ti$ to $Ttot$, is about 40%.

## 4.9 RESPIRATORY THERAPY MODES

Various respiratory therapy modes may be implemented by the disclosed respiratory therapy system.

### 4.9.1 CPAP therapy

In some implementations of respiratory pressure therapy, the central controller 4230 sets the treatment pressure $Pt$ according to the treatment pressure equation (1) as part of the therapy parameter determination algorithm 4329. In one such implementation, the amplitude A is identically zero, so the treatment pressure $Pt$ (which represents a target value to be achieved by the interface pressure $Pm$ at the current instant of time) is identically equal to the base pressure $P_0$ throughout the respiratory cycle. Such implementations are generally grouped under the heading of CPAP therapy. In such implementations, there is no need for the therapy engine module 4320 to determine phase $\Phi$ or the waveform template $\Pi$ ($\Phi$).

In CPAP therapy, the base pressure $P_0$ may be a constant value that is hard-coded or manually entered to the RPT device 4000. Alternatively, the central controller 4230 may repeatedly compute the base pressure $P_0$ as a function of indices or measures of sleep disordered breathing returned by the respective algorithms in the therapy engine module 4320, such as one or more of flow limitation, apnea, hypopnea, patency, and snore. This alternative is sometimes referred to as APAP therapy.

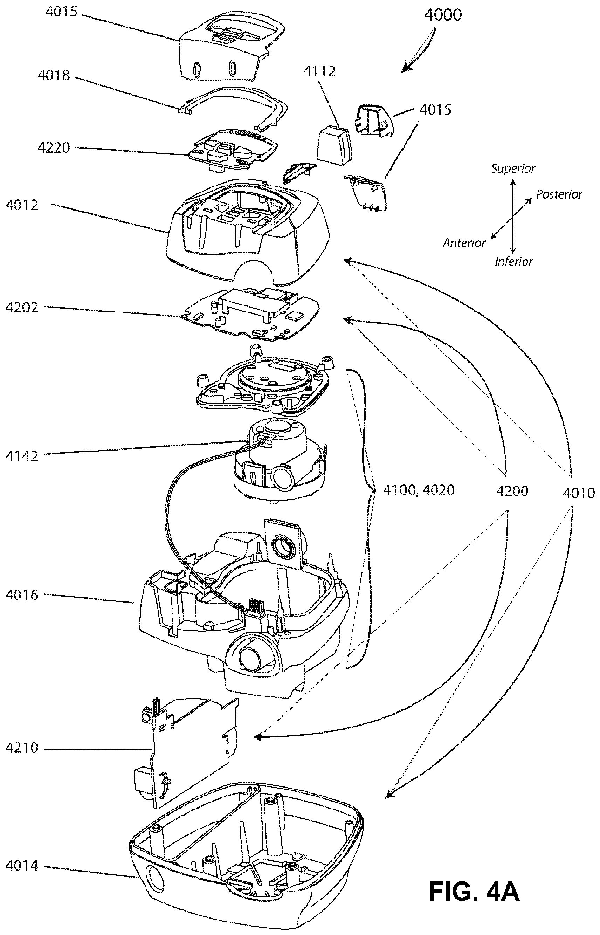

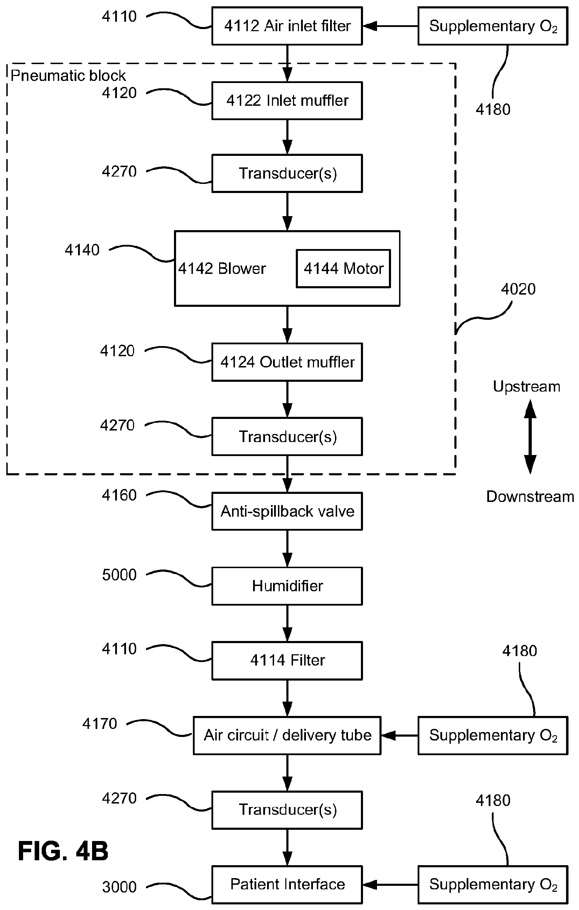

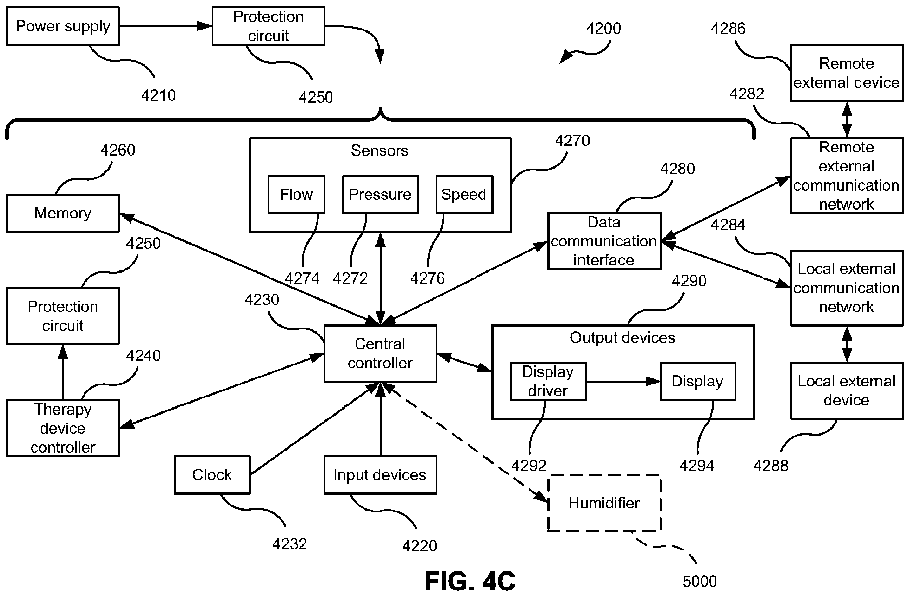

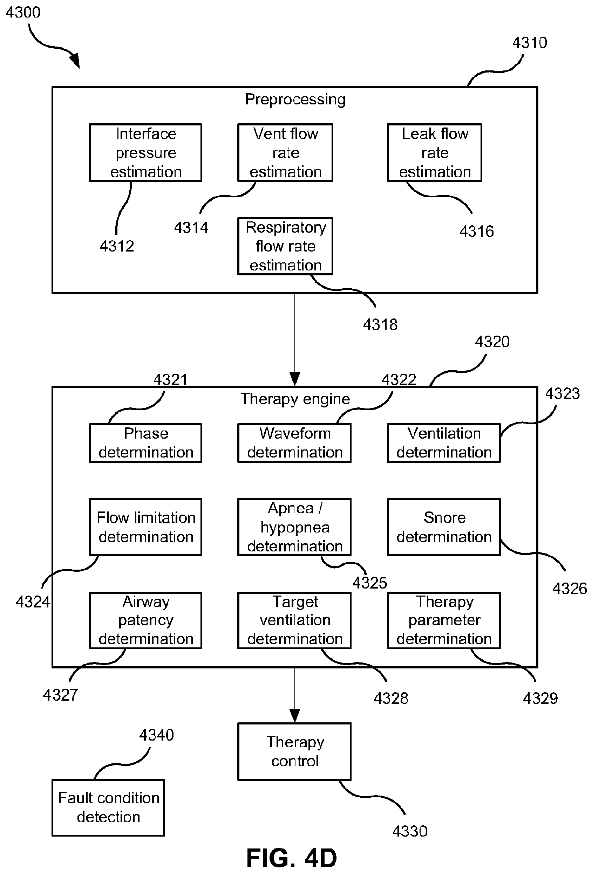

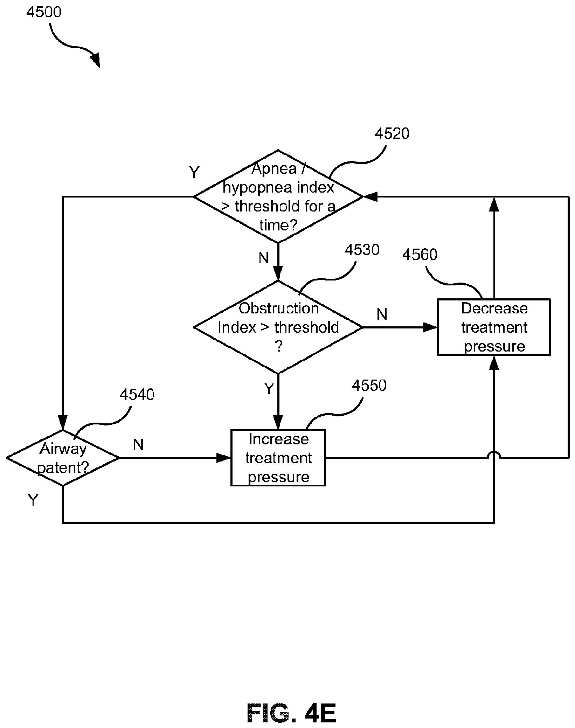

Fig. 4E is a flow chart illustrating a method 4500 carried out by the central controller 4230 to continuously compute the base pressure $P_0$ as part of an APAP therapy implementation of the therapy parameter determination algorithm 4329, when the pressure support A is identically zero.

The method 4500 starts at step 4520, at which the central controller 4230 compares the measure of the presence

**EP 4 387 511 B1**

of apnea / hypopnea with a first threshold, and determines whether the measure of the presence of apnea / hypopnea has exceeded the first threshold for a predetermined period of time, indicating an apnea / hypopnea is occurring. If so, the method 4500 proceeds to step 4540; otherwise, the method 4500 proceeds to step 4530. At step 4540, the central controller 4230 compares the measure of airway patency with a second threshold. If the measure of airway patency exceeds the second threshold, indicating the airway is patent, the detected apnea / hypopnea is deemed central, and the method 4500 proceeds to step 4560; otherwise, the apnea / hypopnea is deemed obstructive, and the method 4500 proceeds to step 4550.

At step 4530, the central controller 4230 compares the measure of flow limitation with a third threshold. If the measure of flow limitation exceeds the third threshold, indicating inspiratory flow is limited, the method 4500 proceeds to step 4550; otherwise, the method 4500 proceeds to step 4560.

At step 4550, the central controller 4230 increases the base pressure $P_0$ by a predetermined pressure increment $\Delta P$, provided the resulting treatment pressure $Pt$ would not exceed a maximum treatment pressure $Pmax$. In one implementation, the predetermined pressure increment $\Delta P$ and maximum treatment pressure $Pmax$ are 1 cmH2O and 25 cmH2O respectively. In other implementations, the pressure increment $\Delta P$ can be as low as 0.1 cmH2O and as high as 3 cmH2O, or as low as 0.5 cmH2O and as high as 2 cmH2O. In other implementations, the maximum treatment pressure $Pmax$ can be as low as 15 cmH2O and as high as 35 cmH2O, or as low as 20 cmH2O and as high as 30 cmH2O. The method 4500 then returns to step 4520.

At step 4560, the central controller 4230 decreases the base pressure $P_0$ by a decrement, provided the decreased base pressure $P_0$ would not fall below a minimum treatment pressure $Pmin$. The method 4500 then returns to step 4520. In one implementation, the decrement is proportional to the value of $P_0$-$Pmin$, so that the decrease in $P_0$ to the minimum treatment pressure $Pmin$ in the absence of any detected events is exponential. In one implementation, the constant of proportionality is set such that the time constant $\tau$ of the exponential decrease of $P_0$ is 60 minutes, and the minimum treatment pressure $Pmin$ is 4 cmH2O. In other implementations, the time constant $\tau$ could be as low as 1 minute and as high as 300 minutes, or as low as 5 minutes and as high as 180 minutes. In other implementations, the minimum treatment pressure $Pmin$ can be as low as 0 cmH2O and as high as 8 cmH2O, or as low as 2 cmH2O and as high as 6 cmH2O. Alternatively, the decrement in $P_0$ could be predetermined, so the decrease in $P_0$ to the minimum treatment pressure $Pmin$ in the absence of any detected events is linear.

### 4.9.2 Bi-level therapy

In other implementations of this form of the present technology, the value of amplitude A in equation (1) may be positive. Such implementations are known as bi-level therapy, because in determining the treatment pressure $Pt$ using equation (1) with positive amplitude A, the therapy parameter determination algorithm 4329 oscillates the treatment pressure $Pt$ between two values or levels in synchrony with the spontaneous respiratory effort of the patient 1000. That is, based on the typical waveform templates $\Pi(\Phi, t)$ described above, the therapy parameter determination algorithm 4329 increases the treatment pressure $Pt$ to $P_0$ + A (known as the IPAP) at the start of, or during, or inspiration and decreases the treatment pressure $Pt$ to the base pressure $P_0$ (known as the EPAP) at the start of, or during, expiration.

In some forms of bi-level therapy, the IPAP is a treatment pressure that has the same purpose as the treatment pressure in CPAP therapy modes, and the EPAP is the IPAP minus the amplitude A, which has a "small" value (a few cmH2O) sometimes referred to as the Expiratory Pressure Relief (EPR). Such forms are sometimes referred to as CPAP therapy with EPR, which is generally thought to be more comfortable than straight CPAP therapy. In CPAP therapy with EPR, either or both of the IPAP and the EPAP may be constant values that are hard-coded or manually entered to the RPT device 4000. Alternatively, the therapy parameter determination algorithm 4329 may repeatedly compute the IPAP and / or the EPAP during CPAP with EPR. In this alternative, the therapy parameter determination algorithm 4329 repeatedly computes the EPAP and / or the IPAP as a function of indices or measures of sleep disordered breathing returned by the respective algorithms in the therapy engine module 4320 in analogous fashion to the computation of the base pressure $P_0$ in APAP therapy described above.

In other forms of bi-level therapy, the amplitude A is large enough that the RPT device 4000 does some or all of the work of breathing of the patient 1000. In such forms, known as pressure support ventilation therapy, the amplitude A is referred to as the pressure support, or swing. In pressure support ventilation therapy, the IPAP is the base pressure $P_0$ plus the pressure support A, and the EPAP is the base pressure $P_0$.

In some forms of pressure support ventilation therapy, known as fixed pressure support ventilation therapy, the pressure support A is fixed at a predetermined value, e.g. 10 cmH2O. The predetermined pressure support value is a setting of the RPT device 4000, and may be set for example by hard-coding during configuration of the RPT device 4000 or by manual entry through the input device 4220.

In other forms of pressure support ventilation therapy, broadly known as servo-ventilation, the therapy parameter determination algorithm 4329 takes as input some currently measured or estimated parameter of the respiratory cycle (e.g. the current measure $Vent$ of ventilation) and a target value of that respiratory parameter (e.g. a target value $Vtgt$ of ventilation) and repeatedly adjusts the parameters of equation (1) to bring the current measure of the respiratory parameter towards the target value. In a form of servo-ventilation known as adaptive servo-ventilation (ASV), which has been used to treat CSR, the respiratory parameter is ventilation, and the target ventilation value *Vtgt* is computed by the target ventilation determination algorithm 4328 from the typical recent ventilation *Vtyp,* as described above.

In some forms of servo-ventilation, the therapy parameter determination algorithm 4329 applies a control methodology to repeatedly compute the pressure support *A* so as to bring the current measure of the respiratory parameter towards the target value. One such control methodology is Proportional-Integral (PI) control. In one implementation of PI control, suitable for ASV modes in which a target ventilation *Vtgt* is set to slightly less than the typical recent ventilation *Vtyp,* the pressure support A is repeatedly computed as:

$$A = G \int (Vent - Vtgt) dt$$

$$(2)$$

where G is the gain of the PI control. Larger values of gain G can result in positive feedback in the therapy engine module 4320. Smaller values of gain G may permit some residual untreated CSR or central sleep apnea. In some implementations, the gain G is fixed at a predetermined value, such as -0.4 cmH2O/(L/min)/sec. Alternatively, the gain G may be varied between therapy sessions, starting small and increasing from session to session until a value that substantially eliminates CSR is reached. Conventional means for retrospectively analysing the parameters of a therapy session to assess the severity of CSR during the therapy session may be employed in such implementations. In yet other implementations, the gain G may vary depending on the difference between the current measure *Vent* of ventilation and the target ventilation *Vtgt*.

Other servo-ventilation control methodologies that may be applied by the therapy parameter determination algorithm 4329 include proportional (P), proportional-differential (PD), and proportional-integral-differential (PID).

The value of the pressure support *A* computed via equation (2) may be clipped to a range defined as [*Amin, Amax*]. In this implementation, the pressure support *A* sits by default at the minimum pressure support *Amin* until the measure of current ventilation *Vent* falls below the target ventilation *Vtgt,* at which point *A* starts increasing, only falling back to *Amin* when *Vent* exceeds *Vtgt* once again.

The pressure support limits *Amin* and *Amax* are settings of the RPT device 4000, set for example by hard-coding during configuration of the RPT device 4000 or by manual entry through the input device 4220.

In pressure support ventilation therapy modes, the EPAP is the base pressure $P_0$. As with the base pressure $P_0$ in CPAP therapy, the EPAP may be a constant value that is prescribed or determined during titration. Such a constant EPAP may be set for example by hard-coding during configuration of the RPT device 4000 or by manual entry through the input device 4220. This alternative is sometimes referred to as fixed-EPAP pressure support ventilation therapy. Titration of the EPAP for a given patient may be performed by a clinician during a titration session with the aid of PSG, with the aim of preventing obstructive apneas, thereby maintaining an open airway for the pressure support ventilation therapy, in similar fashion to titration of the base pressure $P_0$ in constant CPAP therapy.

Alternatively, the therapy parameter determination algorithm 4329 may repeatedly compute the base pressure $P_0$ during pressure support ventilation therapy. In such implementations, the therapy parameter determination algorithm 4329 repeatedly computes the EPAP as a function of indices or measures of sleep disordered breathing returned by the respective algorithms in the therapy engine module 4320, such as one or more of flow limitation, apnea, hypopnea, patency, and snore. Because the continuous computation of the EPAP resembles the manual adjustment of the EPAP by a clinician during titration of the EPAP, this process is also sometimes referred to as auto-titration of the EPAP, and the therapy mode is known as auto-titrating EPAP pressure support ventilation therapy, or auto-EPAP pressure support ventilation therapy.

### 4.10 GLOSSARY

For the purposes of the present technology disclosure, in certain forms of the present technology, one or more of the following definitions may apply. In other forms of the present technology, alternative definitions may apply.

#### 4.10.1 General

*Air*: In certain forms of the present technology, air may be taken to mean atmospheric air, and in other forms of the present technology air may be taken to mean some other combination of breathable gases, e.g. oxygen enriched air.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, $Qd$, is the flow rate of air leaving the RPT device. Total flow rate, $Qt$, is the flow rate of air and any supplementary gas reaching the patient interface via the air circuit. Vent flow rate, $Qv$, is the flow rate of air leaving a vent to allow washout of exhaled gases. Leak flow rate, $Ql$, is the flow rate of leak from a patient interface system or elsewhere. Respiratory flow rate, $Qr$, is the flow rate of air that is received into the patient's respiratory system.

*Flow therapy*: Respiratory therapy comprising the delivery of a flow of air to an entrance to the airways at a controlled flow rate referred to as the treatment flow rate that is typically positive throughout the patient's breathing cycle.

*Humidifier*: The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient by the ambient air. In one form, radiated noise may be quantified by measuring sound power/pressure levels of the object in question according to ISO 3744.

*Noise, vent (acoustic)*: Vent noise in the present document refers to noise which is generated by the flow of air through any vents such as vent holes of the patient interface.

*Oxygen enriched air*: Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen*: Medical oxygen is defined as oxygen enriched air with an oxygen concentration of 80% or *greater. Patient*: A person, whether or not they are suffering from a respiratory condition.

*Pressure:* Force per unit area. Pressure may be expressed in a range of units, including $cmH_2O$, $g$-$f/cm^2$ and hectopascal. 1 cmH2O is equal to 1 $g$-$f/cm^2$ and is approximately 0.98 hectopascal (1 hectopascal = 100 Pa = 100 $N/m^2$ = 1 millibar ~ 0.001 atm). In this specification, unless otherwise stated, pressure is given in units of cmH2O.

The pressure in the patient interface is given the symbol *Pm*, while the treatment pressure, which represents a target value to be achieved by the interface pressure *Pm* at the current instant of time, is given the symbol *Pt*.

*Respiratory Pressure Therapy*: The application of a supply of air to an entrance to the airways at a treatment pressure that is typically positive with respect to atmosphere.

*Ventilator*: A mechanical device that provides pressure support to a patient to perform some or all of the work of breathing.

### 4.10.1.1 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate*: a thermoplastic polymer of Bisphenol-A Carbonate.

### 4.10.1.2 Mechanical properties

*Resilience*: Ability of a material to absorb energy when deformed elastically and to release the energy upon unloading.

*Resilient*: Will release substantially all of the energy when unloaded. Includes e.g. certain silicones, and thermoplastic elastomers.

*Hardness*: The ability of a material per se to resist deformation (e.g. described by a Young's Modulus, or an indentation hardness scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness* (or *rigidity*) of a structure or component: The ability of the structure or component to resist deformation in response to an applied load. The load may be a force or a moment, e.g. compression, tension, bending or torsion. The structure or component may offer different resistances in different directions. The inverse of stiffness is flexibility.

*Floppy* structure or component: A structure or component that will change shape, e.g. bend, when caused to support its own weight, within a relatively short period of time such as 1 second.

*Rigid* structure or component: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH2O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.10.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle*: The ratio of inhalation time, Ti to total breath time, Ttot.

*Effort* (breathing): The work done by a spontaneously breathing person attempting to breathe.

*Expiratory portion* of a breathing cycle: The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:

(i) Flattened: Having a rise followed by a relatively flat portion, followed by a fall.

(ii) M-shaped: Having two local peaks, one at the leading edge, and one at the trailing edge, and a relatively flat portion between the two peaks.

(iii) Chair-shaped: Having a single local peak, the peak being at the leading edge, followed by a relatively flat portion.

(iv) Reverse-chair shaped: Having a relatively flat portion followed by single local peak, the peak being at the trailing edge.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Hyperpnea*: An increase in flow to a level higher than normal.

*Inspiratory portion* of a breathing cycle: The period from the start of inspiratory flow to the start of expiratory flow will be taken to be the inspiratory portion of a breathing cycle.

*Patency* (airway): The degree of the airway being open, or the extent to which the airway is open. A patent airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow rate* ($Qpeak$): The maximum value of flow rate during the inspiratory portion of the respiratory flow waveform.

*Respiratory flow rate, patient airflow rate, respiratory airflow rate* ($Qr$): These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume* ($Vt$): The volume of air inhaled or exhaled during normal breathing, when extra effort is not applied. In principle the inspiratory volume $Vi$ (the volume of air inhaled) is equal to the expiratory volume Ve (the volume of air exhaled), and therefore a single tidal volume $Vt$ may be defined as equal to either quantity. In practice the tidal volume $Vt$ is estimated as some combination, e.g. the mean, of the inspiratory volume Vi and the expiratory volume Ve.

*Inhalation Time* ($Ti$): The duration of the inspiratory portion of the respiratory flow rate waveform.

*Exhalation Time* ($Te$): The duration of the expiratory portion of the respiratory flow rate waveform.

*Total Time* ($Ttot$): The total duration between the start of one inspiratory portion of a respiratory flow rate waveform and the start of the following inspiratory portion of the respiratory flow rate waveform.

*Typical recent ventilation:* The value of ventilation around which recent values of ventilation Vent over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation* ($Vent$): A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.10.3 Ventilation

Adaptive Servo-Ventilator (ASV): A servo-ventilator that has a changeable, rather than fixed target ventilation. The changeable target ventilation may be learned from some characteristic of the patient, for example, a respiratory characteristic of the patient.

Backup rate: A parameter of a ventilator that establishes the minimum breathing rate (typically in number of breaths per minute) that the ventilator will deliver to the patient, if not triggered by spontaneous respiratory effort.

Cycled: The termination of a ventilator's inspiratory phase. When a ventilator delivers a breath to a spontaneously breathing patient, at the end of the inspiratory portion of the breathing cycle, the ventilator is said to be cycled to stop delivering the breath.

Expiratory positive airway pressure (EPAP): a base pressure, to which a pressure varying within the breath is added to produce the desired interface pressure which the ventilator will attempt to achieve at a given time.

End expiratory pressure (EEP): Desired interface pressure which the ventilator will attempt to achieve at the end of the expiratory portion of the breath. If the pressure waveform template $\Pi(\Phi)$ is zero-valued at the end of expiration, i.e. $\Pi$

($\Phi$) = 0 when $\Phi$ = 1, the EEP is equal to the EPAP.

Inspiratory positive airway pressure (IPAP): Maximum desired interface pressure which the ventilator will attempt to achieve during the inspiratory portion of the breath.

Pressure support: A number that is indicative of the increase in pressure during ventilator inspiration over that during ventilator expiration, and generally means the difference in pressure between the maximum value during inspiration and the base pressure (e.g., PS = IPAP - EPAP). In some contexts, pressure support means the difference which the ventilator aims to achieve, rather than what it actually achieves.

Servo-ventilator: A ventilator that measures patient ventilation, has a target ventilation, and which adjusts the level of pressure support to bring the patient ventilation towards the target ventilation.

Spontaneous/Timed (S/T): A mode of a ventilator or other device that attempts to detect the initiation of a breath of a spontaneously breathing patient. If however, the device is unable to detect a breath within a predetermined period of time, the device will automatically initiate delivery of the breath.

Swing: Equivalent term to pressure support.

Triggered: When a ventilator, or other respiratory therapy device such as an RPT device or portable oxygen concentrator, delivers a volume of breathable gas to a spontaneously breathing patient, it is said to be triggered to do so. Triggering usually takes place at or near the initiation of the respiratory portion of the breathing cycle by the patient's efforts.

### 4.10.3.1 Anatomy of the respiratory system

Diaphragm: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

Larynx: The larynx, or voice box houses the vocal folds and connects the inferior part of the pharynx (hypopharynx) with the trachea.

Lungs: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

Nasal cavity: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

Pharynx: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.10.4 Patient interface

Anti-asphyxia valve (AAV): The component or sub-assembly of a mask system that, by opening to atmosphere in a failsafe manner, reduces the risk of excessive $CO_2$ rebreathing by a patient.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

Headgear: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use.

A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

## 4.11 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

4.12 LIST OF SELECTED REFERENCE SIGNS

| patient | 1000 |
| --- | --- |
| bed partner | 1100 |
| patient interface | 3000 |
| seal forming structure | 3100 |
| plenum chamber | 3200 |
| positioning and stabilising structure | 3300 |
| vent | 3400 |
| connection port | 3600 |
| forehead support | 3700 |
| patient interface | 3800 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| air filter | 4110 |
| inlet air filter | 4112 |
| muffler | 4120 |
| inlet muffler | 4122 |
| outlet muffler | 4124 |
| pressure generator | 4140 |
| blower | 4142 |
| motor | 4144 |
| anti-spillback valve | 4160 |
| air circuit | 4170 |
| supplementary gas | 4180 |
| electrical components | 4200 |
| PCBA | 4202 |
| electrical power supply | 4210 |
| input device | 4220 |

(continued)

| | |
|---|---|
| central controller | 4230 |
| clock | 4232 |
| therapy device controller | 4240 |
| protection circuit | 4250 |
| memory | 4260 |
| transducers | 4270 |
| pressure sensor | 4272 |
| flow rate sensor | 4274 |
| motor speed transducer | 4276 |
| data communication interface | 4280 |
| remote external communication network | 4282 |
| local external communication network | 4284 |
| remote external device | 4286 |
| local external connection | 4288 |
| output device | 4290 |
| display driver | 4292 |
| display | 4294 |
| algorithms | 4300 |
| pre-processing module | 4310 |
| interface pressure estimation algorithm | 4312 |
| vent flow rate estimation algorithm | 4314 |
| leak flow rate estimation algorithm | 4316 |
| respiratory flow rate estimation algorithm | 4318 |
| therapy engine module | 4320 |
| phase determination algorithm | 4321 |
| waveform algorithm | 4322 |
| ventilation determination algorithm | 4323 |
| inspiratory flow limitation determination algorithm | 4324 |
| apnea / hypopnea determination algorithm | 4325 |
| snore determination algorithm | 4326 |
| airway patency determination algorithm | 4327 |
| target ventilation determination algorithm | 4328 |
| therapy parameter determination algorithm | 4329 |
| therapy control module | 4330 |
| methods | 4340 |
| methods | 4500 |
| step | 4520 |
| step | 4530 |
| step | 4540 |
| step | 4550 |

(continued)

| step | 4560 |
|---|---|
| humidifier | 5000 |
| humidifier inlet | 5002 |
| humidifier outlet | 5004 |
| humidifier base | 5006 |
| humidifier reservoir | 5110 |
| conductive portion | 5120 |
| reservoir dock | 5130 |
| locking lever | 5135 |
| water level indicator | 5150 |
| heating element | 5240 |
| valve assembly | 6000 |
| valve housing | 6010 |
| upstream housing portion | 6012 |
| downstream housing portion | 6014 |
| pressure activated valve member | 6020 |
| inlet/RPT port | 6030 |
| outlet/patient interface port | 6040 |
| vent/ambient port | 6050 |
| vent opening | 6052 |
| diaphragm | 6060 |
| outer circumference | 6070 |
| retention flange | 6080 |
| one way / duck bill valve | 6090 |
| membrane portion | 6100 |
| membrane section/portion | 6102 |
| pressure sensor (patient interface) | 6110 |
| recess | 6120 |
| rib | 6130 |
| first wall | 6140 |
| lips | 6150 |
| ends of lips | 6160 |
| opening | 6170 |
| flow guide portion | 6180 |
| ramp | 6190 |
| side wall | 6200 |
| upper edge | 6210 |
| chamber | 6220 |
| HMX | 7000 |
| hygroscopic material | 7010 |

(continued)

| frame members | 7020 |
|---|---|
| length of base of valve | L |
| Width of base of valve | W |

**Claims**

1. A respiratory treatment system for delivering pressurised air to an entrance to a patient's airways, the system being configured to maintain a therapy pressure in a range suitable for treating respiratory disorders, the respiratory treatment system comprising at least:

   a. a blower for generating the pressurised air;
   b. a patient interface (300) for sealing delivery of the pressurised air to the patient airways; and
   c. a combined one-way inspiratory valve and expiratory release valve assembly (6000) for controlling air flow to the patient interface (3000),

   the combined valve assembly (6000) comprising:

   a housing (6010) comprising a valve inlet (6030), a valve outlet (6040) and at least one vent opening (6052),
   a diaphragm (6060) sealingly connected to the housing (6010) at an outer circumference (6070) of the diaphragm (6060), wherein the diaphragm (6060) divides the housing (6010) into; a) an upstream portion (6012), which is in fluid communication with the valve inlet (6030), and b) a downstream portion (6014) which is in fluid communication with the valve outlet (6040),

   wherein the diaphragm (6060) has an oval, elliptical or stadium shape,
   wherein an inner portion of the diaphragm (6060) defines a one-way inspiratory valve configured to;

   - allow flow from the valve inlet (6030) to the valve outlet (6040) when the pressure in the upstream portion (6012) of the housing (6010) exceeds the pressure in the downstream portion (6014) of the housing (6010); and
   - reduce or substantially prevent flow from the valve inlet (6030) to the valve outlet (6040) when the pressure in the downstream portion (6014) is greater than the pressure in the upstream portion (6012),

   wherein an outer portion of the diaphragm (6060) defines an expiratory release valve that,

   - when the pressure in the downstream portion (6014) exceeds the pressure in the upstream portion (6012), allows flow from the downstream portion (6014) of the housing (6010) to ambient atmosphere via the at least one vent opening (6052); and
   - when the pressure in the upstream portion (6012) exceeds the pressure in the downstream portion (6014), reduces or substantially prevents flow from the downstream portion (6014) of the housing (6010) to ambient atmosphere via the at least one vent opening (6052),

   the respiratory treatment system further comprising a pressure sensor (6110) configured to measure pressure within the patient interface, wherein the system controls the blower based on data from the pressure sensor (6110),
   wherein the system reduces flow from the blower when the pressure sensor (6110) detects that the patient is exhaling.

2. The respiratory treatment system of claim 1, wherein the diaphragm (6060) has an elongate shape, and a ratio of the length of the major axis of the diaphragm (6060) to the length of the minor axis of the diaphragm (6060) is at least 4:3.

3. The respiratory treatment system of claim 2, wherein the one-way inspiratory valve is configured as a duckbill valve (6090), and wherein a base of the duckbill valve (6090) has a length dimension substantially parallel to the major axis of the diaphragm (6060) and a width dimension substantially parallel to the minor axis of the diaphragm (6060), wherein the length is greater than the width, and wherein the ratio of length to width is at least 1.5:1.

4. The respiratory treatment system of any one of claims 1 to 3, wherein the diaphragm (6060) comprises a cylindrical wall (6140) and a retention flange (6080) provided to one end of the cylindrical wall (6140).

5. The respiratory treatment system of any one of claims 1 to 4, wherein a first portion of the diaphragm (6060) is made from a different material to an adjacent portion.

6. The respiratory treatment system of any one of claims 1 to 5, wherein the diaphragm (6060) has regions of differing thickness, wherein one of the regions has a thickness which is at least twice the thickness of another of the regions.

7. The respiratory treatment system of claim 6, wherein the one-way inspiratory valve has a first region having a first thickness and a second region, adjacent the first region, having a second thickness.

8. The respiratory treatment system of claim 6 or 7, wherein the expiratory release valve has a first region having a first thickness and a second region, adjacent the first region, having a second thickness.

9. The respiratory treatment system of any one of claims 1 to 8, wherein the diaphragm (6060) has no more than a single axis of symmetry.

10. The respiratory treatment system of any one of claims 1 to 9, wherein the downstream portion (6014) of the housing (6010) comprises the valve outlet (6040), wherein the diaphragm (6060) comprise a pair of lips (6150) that are pushed towards each other to seal the pathway to the patient interface when the pressure in the downstream portion (6014) exceeds the pressure in the upstream portion (6012), and wherein the pair of lips (6150) create an opening to allow airflow to pass through towards the patient interface entrance, when the pressure in the upstream portion (6012) of the housing (6010) exceeds the pressure in the downstream portion (6014).

11. The respiratory treatment system of claim 10, wherein a flow guide (6180) is provided for each vent opening (6052), each flow guide (6180) configured to avoid or minimise at least one of a) sharp corners; b) sharp angles; and c) sudden expansion, of expiratory gases flowing from the valve outlet (6040) to the respective vent opening (6052), when the pressure in the downstream portion (6014) exceeds the pressure in the upstream portion (6012).

12. The respiratory treatment system of any one of claims 1 to 11, wherein the system comprises a portable integrated blower/patent interface system wearable on the patient's face or head.

**Patentansprüche**

1. Atembehandlungssystem zum Zuführen von Druckluft zu einem Eingang der Atemwege eines Patienten, wobei das System dafür konfiguriert ist, einen Therapiedruck in einem für die Behandlung von Atemwegserkrankungen geeigneten Bereich aufrechtzuerhalten, wobei das Atembehandlungssystem mindestens aufweist:

   a. ein Gebläse zum Erzeugen der Druckluft;
   b. eine Patientenschnittstelle (300) zum Abdichten der Zufuhr der Druckluft zu den Atemwegen des Patienten; und
   c. eine kombinierte Einwege-Inspirationsventil- und Expirationsablassventilanordnung (6000) zum Steuern des Luftstroms zur Patientenschnittstelle (3000),

      wobei die kombinierte Ventilanordnung (6000) aufweist:

         ein Gehäuse (6010) mit einem Ventileinlass (6030), einem Ventilauslass (6040) und mindestens einer Entlüftungsöffnung (6052);
         eine Membran (6060), die an einem Außenumfang (6070) der Membran (6060) dichtend mit dem Gehäuse (6010) verbunden ist, wobei die Membran (6060) das Gehäuse (6010) unterteilt in: a) einen stromaufwärtsseitigen Abschnitt (6012), der in Fluidverbindung mit dem Ventileinlass (6030) steht, und b) einen stromabwärtsseitigen Abschnitt (6014), der in Fluidverbindung mit dem Ventilauslass (6040) steht,

wobei die Membran (6060) eine ovale, elliptische oder stadionförmige Konfiguration aufweist,

wobei ein innerer Abschnitt der Membran (6060) ein Einwege-Inspirationsventil definiert, das dafür konfiguriert ist,

einen Durchfluss vom Ventileinlass (6030) zum Ventilauslass (6040) zu ermöglichen, wenn der Druck im stromaufwärtsseitigen Abschnitt (6012) des Gehäuses (6010) den Druck im stromabwärtsseitigen Abschnitt (6014) des Gehäuses (6010) übersteigt; und

den Durchfluss vom Ventileinlass (6030) zum Ventilauslass (6040) zu reduzieren oder im Wesentlichen zu verhindern, wenn der Druck im stromabwärtsseitigen Abschnitt (6014) größer ist als der Druck im stromaufwärtsseitigen Abschnitt (6012),

wobei ein äußerer Abschnitt der Membran (6060) ein Expirationsablassventil definiert, das

wenn der Druck im stromabwärtsseitigen Abschnitt (6014) den Druck im stromaufwärtsseitigen Abschnitt (6012) übersteigt, einen Durchfluss vom stromabwärtsseitigen Abschnitt (6014) des Gehäuses (6010) über die mindestens eine Entlüftungsöffnung (6052) zur Umgebungsatmosphäre ermöglicht; und

wenn der Druck im stromaufwärtsseitigen Abschnitt (6012) den Druck im stromabwärtsseitigen Abschnitt (6014) übersteigt, den Durchfluss vom stromabwärtsseitigen Abschnitt (6014) des Gehäuses (6010) über die mindestens eine Entlüftungsöffnung (6052) zur Umgebungsatmosphäre reduziert oder im Wesentlichen verhindert,

wobei das Atembehandlungssystem ferner einen Drucksensor (6110) aufweist, der dafür konfiguriert ist, den Druck innerhalb der Patientenschnittstelle zu messen, wobei das System das Gebläse basierend auf Daten vom Drucksensor (6110) steuert,

wobei das System den Durchfluss vom Gebläse reduziert, wenn der Drucksensor (6110) erfasst, dass der Patient ausatmet.

2. Atembehandlungssystem nach Anspruch 1, wobei die Membran (6060) eine längliche Form aufweist und das Verhältnis zwischen der Länge der Hauptachse der Membran (6060) und der Länge der Nebenachse der Membran (6060) mindestens 4:3 beträgt.

3. Atembehandlungssystem nach Anspruch 2, wobei das Einwege-Inspirationsventil als Entenschnabelventil (6090) ausgebildet ist, und wobei eine Basis des Entenschnabelventils (6090) eine Längenabmessung aufweist, die sich im Wesentlichen parallel zur Hauptachse der Membran (6060) erstreckt, und eine Breitenabmessung, die sich im Wesentlichen parallel zur Nebenachse der Membran (6060) erstreckt, wobei die Länge größer ist als die Breite und wobei das Verhältnis zwischen Länge und Breite mindestens 1,5:1 beträgt.

4. Atembehandlungssystem nach einem der Ansprüche 1 bis 3, wobei die Membran (6060) eine zylindrische Wand (6140) und einen an einem Ende der zylindrischen Wand (6140) vorgesehenen Halteflansch (6080) aufweist.

5. Atembehandlungssystem nach einem der Ansprüche 1 bis 4, wobei ein erster Abschnitt der Membran (6060) aus einem anderen Material hergestellt ist als ein benachbarter Abschnitt.

6. Atembehandlungssystem nach einem der Ansprüche 1 bis 5, wobei die Membran (6060) Bereiche unterschiedlicher Dicke aufweist, wobei einer der Bereiche eine Dicke aufweist, die mindestens die doppelte Dicke eines anderen der Bereicheaufweist.

7. Atembehandlungssystem nach Anspruch 6, wobei das Einwege-Inspirationsventil einen ersten Bereich mit einer ersten Dicke und einen dem ersten Bereich benachbarten zweiten Bereich mit einer zweiten Dicke aufweist.

8. Atembehandlungssystem nach Anspruch 6 oder 7, wobei das Expirationsablassventil einen ersten Bereich mit einer ersten Dicke und einen dem ersten Bereich benachbarten zweiten Bereich mit einer zweiten Dicke aufweist.

9. Atembehandlungssystem nach einem der Ansprüche 1 bis 8, wobei die Membran (6060) nicht mehr als eine einzelne Symmetrieachse aufweist.

10. Atembehandlungssystem nach einem der Ansprüche 1 bis 9, wobei der stromabwärtsseitige Abschnitt (6014) des Gehäuses (6010) den Ventilauslass (6040) aufweist, wobei die Membran (6060) ein Paar Lippen (6150) aufweist, die gegeneinander gedrückt werden, um den Weg zur Patientenschnittstelle abzudichten, wenn der Druck im stromabwärtsseitigen Abschnitt (6014) den Druck im stromaufwärtsseitigen Abschnitt (6012) übersteigt, und wobei das Paar Lippen (6150) eine Öffnung bildet, um einen Luftstrom in Richtung zum Eingang der Patientenschnittstelle durchzulassen, wenn der Druck im stromaufwärtsseitigen Abschnitt (6012) des Gehäuses (6010) den Druck im stromabwärtsseitigen Abschnitt (6014) übersteigt.

11. Atembehandlungssystem nach Anspruch 10, wobei für jede Entlüftungsöffnung (6052) eine Strömungsführung (6180) vorgesehen ist, wobei jede Strömungsführung (6180) derart konfiguriert ist, dass mindestens eines der folgenden Merkmale vermieden oder minimiert wird: a) scharfe Ecken; b) scharfe Winkel und c) plötzliche Ausdehnung der vom Ventilauslass (6040) zur jeweiligen Entlüftungsöffnung (6052) strömenden Expirationsgase, wenn der Druck im stromabwärtsseitigen Abschnitt (6014) den Druck im stromaufwärtsseitigen Abschnitt (6012) übersteigt.

12. Atembehandlungssystem nach einem der Ansprüche 1 bis 11, wobei das System ein tragbares integriertes Gebläse-/Patientenschnittstellensystem aufweist, das am Gesicht oder Kopf des Patienten getragen werden kann.

**Revendications**

1. Système de traitement respiratoire pour l'administration d'air sous pression à une entrée des voies respiratoires d'un patient, le système étant configuré pour maintenir une pression thérapeutique dans une plage appropriée au traitement des troubles respiratoires, le système de traitement respiratoire comprenant au moins:

   a. un ventilateur pour générer l'air sous pression;
   b. une interface patient (300) pour l'administration étanche de l'air sous pression aux voies respiratoires du patient; et
   c. un ensemble valve inspiratoire unidirectionnelle et valve de décharge expiratoire combiné (6000) pour commander le flux d'air vers l'interface patient (3000),
   l'ensemble de valves combiné (6000) comprenant:

   un boîtier (6010) comprenant une entrée de valve (6030), une sortie de valve (6040) et au moins une ouverture de ventilation (6052),
   un diaphragme (6060) relié de manière étanche au boîtier (6010) au niveau d'une circonférence extérieure (6070) du diaphragme (6060), où le diaphragme (6060) divise le boîtier (6010) en: a) une partie amont (6012), qui est en communication fluidique avec l'entrée de valve (6030), et b) une partie aval (6014) qui est en communication fluidique avec la sortie de valve (6040),

   où le diaphragme (6060) a une forme ovale, elliptique ou de stade,
   où une partie intérieure du diaphragme (6060) définit une valve inspiratoire unidirectionnelle configurée pour:

   - permettre un flux de l'entrée de valve (6030) à la sortie de valve (6040) lorsque la pression dans la partie amont (6012) du boîtier (6010) dépasse la pression dans la partie aval (6014) du boîtier (6010); et
   - réduire ou empêcher sensiblement le flux de l'entrée de valve (6030) à la sortie de valve (6040) lorsque la pression dans la partie aval (6014) est supérieure à la pression dans la partie amont (6012),

   où une partie extérieure du diaphragme (6060) définit une valve de décharge expiratoire qui,

   - lorsque la pression dans la partie aval (6014) dépasse la pression dans la partie amont (6012), permet un flux de la partie aval (6014) du boîtier (6010) à l'atmosphère ambiante via la au moins une ouverture de ventilation (6052); et
   - lorsque la pression dans la partie amont (6012) dépasse la pression dans la partie aval (6014), réduit ou empêche sensiblement le flux de la partie aval (6014) du boîtier (6010) à l'atmosphère ambiante via la au moins une ouverture de ventilation (6052),
   le système de traitement respiratoire comprenant en outre un capteur de pression (6110) configuré pour mesurer la pression dans l'interface patient, où le système commande le ventilateur sur la base de données provenant du capteur de pression (6110),
   où le système réduit le flux provenant du ventilateur lorsque le capteur de pression (6110) détecte que le patient expire.

2. Système de traitement respiratoire selon la revendication 1, où le diaphragme (6060) a une forme allongée, et un rapport de la longueur du grand axe du diaphragme (6060) à la longueur du petit axe du diaphragme (6060) est d'au moins 4:3.

3. Système de traitement respiratoire selon la revendication 2, où la valve inspiratoire unidirectionnelle est configurée comme une valve à bec de canard (6090), et où une base de la valve à bec de canard (6090) a une dimension de la longueur sensiblement parallèle au grand axe du diaphragme (6060) et une dimension de la largeur sensiblement parallèle au petit axe du diaphragme (6060), où la longueur est supérieure à la largeur, et où le rapport de la longueur à la largeur est d'au moins 1,5:1.

4. Système de traitement respiratoire selon l'une quelconque des revendications 1 à 3, où le diaphragme (6060) comprend une paroi cylindrique (6140) et une bride de retenue (6080) prévue à une extrémité de la paroi cylindrique (6140).

5. Système de traitement respiratoire selon l'une quelconque des revendications 1 à 4, où une première partie du diaphragme (6060) est faite d'un matériau différent de celui d'une partie adjacente.

6. Système de traitement respiratoire selon l'une quelconque des revendications 1 à 5, où le diaphragme (6060) a des régions d'épaisseur différente, où l'une des régions a une épaisseur qui est au moins deux fois l'épaisseur d'une autre des régions.

7. Système de traitement respiratoire selon la revendication 6, où la valve inspiratoire unidirectionnelle a une première région ayant une première épaisseur et une seconde région, adjacente à la première région, ayant une seconde épaisseur.

8. Système de traitement respiratoire selon la revendication 6 ou 7, où la valve de décharge expiratoire a une première région ayant une première épaisseur et une seconde région, adjacente à la première région, ayant une seconde épaisseur.

9. Système de traitement respiratoire selon l'une quelconque des revendications 1 à 8, où le diaphragme (6060) n'a pas plus qu'un seul axe de symétrie.

10. Système de traitement respiratoire selon l'une quelconque des revendications 1 à 9, où la partie aval (6014) du boîtier (6010) comprend la sortie de valve (6040), où le diaphragme (6060) comportant une paire de lèvres (6150) qui sont poussées l'une vers l'autre pour obturer le passage vers l'interface patient lorsque la pression dans la partie aval (6014) dépasse la pression dans la partie amont (6012), et où la paire de lèvres (6150) crée une ouverture pour permettre au flux d'air de traverser vers l'entrée de l'interface patient lorsque la pression dans la partie amont (6012) du boîtier (6010) dépasse la pression dans la partie aval (6014).

11. Système de traitement respiratoire selon la revendication 10, où un guide de flux (6180) est prévu pour chaque ouverture de ventilation (6052), chaque guide de flux (6180) configuré pour éviter ou minimiser au moins l'un de: a) les coins aigus; b) les angles aigus; et c) l'expansion subite des gaz expirés s'écoulant de la sortie de valve (6040) à l'ouverture de ventilation (6052) respective, lorsque la pression dans la partie aval (6014) dépasse la pression dans la partie amont (6012).

12. Système de traitement respiratoire selon l'une quelconque des revendications 1 à 11, où le système comprend un système ventilateur/interface patient intégré portable pouvant être porté sur le visage ou la tête du patient.

FIG. 1A

**FIG. 1B**

FIG. 1C

**FIG. 2A**

**FIG. 3A**

4015

4018

4220

4112

4000

4015

4012

Superior

Posterior

Anterior

Inferior

4202

4142

4100, 4020

4200

4010

4016

4210

4014

**FIG. 4A**

**FIG. 4B**

FIG. 4C

4300

4310

Preprocessing

| Interface pressure estimation | Vent flow rate estimation | | Leak flow rate estimation |

4312

4314

Respiratory flow rate estimation

4316

4318

4320

Therapy engine

4321

4322

4323

| Phase determination | Waveform determination | Ventilation determination |

| Flow limitation determination | Apnea / hypopnea determination | Snore determination |

4324

4325

4326

| Airway patency determination | Target ventilation determination | Therapy parameter determination |

4327

4328

4329

4340

Therapy control

Fault condition detection

4330

**FIG. 4D**

4500

4520

Y ← Apnea /
hypopnea index
> threshold for a
time?

N

4530    4560

Obstruction
Index > threshold
?    → N →    Decrease
treatment
pressure

Y

4540

4550

Airway
patent?    → N →    Increase
treatment
pressure

Y

**FIG. 4E**

**FIG. 5A**

**FIG. 5B**

**FIG. 6**

EP 4 387 511 B1

FIG. 7

FIG. 8

FIG. 9

FIG. 10

6150

6080

6070

6020

6090

6150

## FIG. 11

6150

6140

6080

## FIG. 12

P$_2$

P$_1$

FIG. 13

FIG. 14

FIG. 15

6100

6130

Pressure
sensor

6110

6040

FIG. 16

FIG. 17

FIG. 18

FIG. 19

FIG. 20

FIG. 21

Fig. 22

**Fig. 23**

FIG. 24

**FIG. 25**

EAV  Characterisation_Flow Diagram

Characterised EAV
venting flow rate, called
$Q_V$

FG
Known flow rate,
called $Q_B$

Mask System

Patient
Unknown flow rate,
called $Q_P$

Unknown unintentional
leak flow rate, called $Q_L$

$$Q_P = Q_B - Q_V - Q_L$$

**Fig. 26**

3000

6000

4170

FIG. 27

**Fig. 28**

EP 4 387 511 B1

**Fig. 29**

**Fig. 30**

**Fig. 31**

FIG. 32

FIG. 33

FIG. 34

FIG. 35

FIG. 36

FIG. 37

FIG. 38

FIG. 39

FIG. 40

Performing at least one simulated breathing cycle with the respiratory treatment system including the valve

↓

During the at least one simulated breathing cycle, measuring the flow rate through the valve vent, the pressure upstream of the valve and the pressure downstream of the valve

↓

Plotting vent flow rate against a ratio of the pressures on both sides of the valve diaphragm

↓

Identifying if there are any boundary points dividing the plotted data into one or more contiguous zones according to trends in the data

↓

Fitting equations for best fit curves for the data in each of the identified zones

↓

Deriving, from the fitted equations, at least the coefficients and constants characterizing the respective function between the pressure ratio and the vent flow for each zone, for the first treatment pressure

↓

Repeating the above steps to derive coefficients and constants for other treatment pressures

↓

Interpolating the derived coefficients and constants to derive coefficients and constants for yet further treatment pressures

↓

Using the derived coefficients and constants to, for a given ratio of the pressures on both sides of the valve, calculate a respective vent flow, for a respective treatment pressure

**Fig. 41**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4944310 A, Sullivan
- US 6532959 B, Berthon-Jones
- WO 1998034665 A
- WO 2000078381 A
- US 6581594 B
- US 20090050156 A
- US 20090044808
- WO 2011051908 A1

- US 6634357 B1
- US 2021038849 A1
- US 7866944 B
- US 8638014 B
- US 8636479 B
- WO 2013020167 A
- US 8733349 B

**Non-patent literature cited in the description**

- **JOHN B. WEST**. Respiratory Physiology''. Lippincott Williams & Wilkins, 2012